# EUROPEAN PATENT SPECIFICATION

(11) **EP 4 228 738 B1**
(45) Date of publication and mention of the grant of the patent: **19.08.2026**
(21) Application number: 21820130.9
(22) Date of filing: 16.11.2021
(51) Int. Cl.: A61N 1/04, A61N 1/36

(54) **SYSTEM FOR TREATING CERVICAL DYSTONIA**
SYSTEM ZUR BEHANDLUNG VON ZERVIXDYSTONIE
SYSTÈME DE TRAITEMENT DE LA DYSTONIE CERVICALE

(30) Priority: 16.11.2020 EP 20207938
(43) Date of publication of application: 23.08.2023
(73) Proprietor: MED-EL Elektromedizinische Geräte GmbH, 6020 Innsbruck (AT)
(72) Inventor: LINDENTHALER, Werner, 6020 Innsbruck (AT); RUBIOLO, Cristina, 1210 Wien (AT); VARGA LUNA, Jose Luis, 6020 Innsbruck (AT)
(74) Representative: Lucke, Andreas
(86) International application number: PCT/EP2021/081906
(87) International publication number: WO 2022/101520

(56) References cited:
- EP-A1- 2 830 703
- WO-A1-2008/131941
- WO-A1-2011/067327
- US-A1- 2009 319 003

## Description

### FIELD OF THE INVENTION

The present invention is in the field of medical devices. More precisely, the present invention relates to an electrostimulation system for the treatment of cervical dystonia.

### BACKGROUND

Cervical dystonia, also known as spasmodic torticollis, is a neurological disorder characterized by involuntary muscle contractions in the neck that cause abnormal movements and postures of the neck and head.

In some cases of cervical dystonia, abnormal contractions may be sustained or continuous; in others, they may be present as spasms that can resemble tremor. The severity of cervical dystonia can vary, but the disorder generally causes significant pain and discomfort as well as difficulties due to the abnormal postures. The symptoms of cervical dystonia may begin slowly and can involve any of the muscles of the neck. The head posture in cervical dystonia can vary. The most common abnormal posture associated with cervical dystonia is the twisting of the chin toward a shoulder so that the head rotates sideways (torticollis). Other abnormal postures associated with cervical dystonia include anterocollis, in which the head tips forward; retrocollis, in which the head is tilted backward; or laterocollis, in which the head tilts toward one side. There can also be shifting of the head on the shoulders in a forward (anterior sagittal shift) or backward (posterior sagittal shift) direction. Often cervical dystonia is complex and combines several angles of head movement. In some people with isolated cervical dystonia, there may also be postural tremor of the hands.

There can be secondary problems arising from cervical dystonia that include cervical spine arthritis, compression of nerve roots, and sometimes narrowing of the spinal cord in the neck (cervical stenosis). Pain directly related to cervical dystonia typically is on the same side as the head turn and is felt as muscular pain in the area of the overactive muscles. Pain can potentially become severe and disabling.

Nowadays there are essentially three treatment options: Botulinum toxin injections, oral medication, and surgical intervention.

Botulinum toxin injections are the treatment of choice for focal dystonia, which is the neck and head in cervical dystonia. Botulinum toxin is a neurotoxin that is injected into the dystonic neck muscles in small doses. Botulinum toxin works by preventing the nerve from releasing acetylcholine, that elicits muscle contractions, and thus causes weakness of the muscle. The effect of botulinum toxin on the muscle begins approximately 2-3 days following injection, peaks at about 4 weeks, and provides relief for approximately 2-6 months.

Oral medications, such as dopaminergic agents (levodopa), anticholinergic agents (benztropine, trihexyphenidyl), baclofen, and clonazepam, can be effective in treating generalized dystonia, in particular in children. In adults, the side effects of these agents, including memory problems and sedation, often occur before an effective dose can be reached.

Surgical interventions for treating cervical dystonia can include selective peripheral denervation, foreseeing the severing of the nerves to the dystonic muscles, and deep brain stimulation surgery (DBS).

Deep brain stimulation surgery (DBS) can be effective for cervical dystonia and may be appropriate for patients who lose botulinum toxin response or have a form of cervical dystonia that is difficult to treat with injections, in particular anterocollis. DBS involves the placement of electrodes (thin wires) into the area of the brain called the globus pallidus on both sides. The electrodes are connected to stimulators, which send small electrical pulses to the brain. Although the precise mechanism is not clear, the electrical impulses seem to "reset" the brain and improve the dystonic movements. After the DBS is placed, the stimulators may be programmed for the optimal outcome.

US20090319003 discloses an electrical stimulation device and method for the reduction of joint compression. The method utilizes an electrical stimulation device that includes a plurality of channels of electrodes each of which includes at least a first and second electrode positioned in electrical contact with tissue of at least two muscles crossing a joint. Agonist/antagonist muscles involved in abduction/adduction, flexion/extension, supination/pronation, protraction/retraction, and/or eversion/inversion of body regions via joint movement are stimulated with a patterned series of electrical pulses through channels of electrodes in accordance with a procedure for reducing joint compression.

WO2011067327 discloses a method and device for muscle relaxation. More specifically, the invention relates to a system that causes muscle relaxation by reducing muscular spasticity through the stimulation of joints and muscles. The system consists of a garment with electrodes, a hardware unit and software controlling the stimulation.

In addition, physical therapy may provide a helpful complement to medical treatment.

### SUMMARY OF THE INVENTION

The invention is defined by the independent claim 1.

However, the known cervical dystonia therapies are merely symptomatic as they exclusively focus on the relief of spasms, pain, and aberrant postures or functions.

For example, botulinum toxin treatment is not a cure, but rather a symptom treatment. When the effect of botulinum toxin wears off, the symptoms of cervical dystonia recur, and another injection is needed.

Although being reported to be effective, the severing of the nerves to the dystonic muscles is limited by the ability to access the nerve involved, the need for considerable expertise of the surgeon, the potential for side effects, which are not uncommon for this procedure, and the need of a long period of rehabilitation. Similarly, deep brain stimulation is associated with a complicated surgical intervention to the brain and therefore suffers from the same shortcomings.

**In** view of this state-of-the-art, the object of the invention is to provide a treatment for cervical dystonia overcoming the shortcomings of the prior art.

This object is solved by a system according to claim 1. The dependent claims relate to preferred embodiments.

According to a first aspect, the invention relates to a system for treating cervical dystonia, wherein the system comprises a first electrode and a stimulator. The first electrode is placed or adapted to be placed in functional proximity to at least one of an agonist muscle and an antagonist muscle and/or their innervating nerves. The stimulator is coupled to the first electrode and is configured to apply a pulsed electrical stimulation signal to the first electrode, wherein the pulsed electrical stimulation signal is configured for selectively increasing or reducing the activity of the at least one of the agonist muscle and the antagonist muscle, wherein the pulsed electrical stimulation signal is adapted to reduce an imbalance in the activity of the agonist muscle and the antagonist muscle.

The invention was developed based on the working hypothesis of the inventors that in cervical dystonia, the head, neck, and/or upper chest posture is impaired because of the deficit in coordination between agonist and antagonist muscle(s) involved in the onset of the assessed cervical dystonia type (or in the following in short the agonist(s) and antagonist(s)). In this disease the agonistic muscle(s) show hypo-functionality and/or the antagonistic muscle(s) hyper-functionality, with respect to a healthy state, in which both agonist and antagonist functions are counteracting each other but at the same time also balancing each other. In other words, the terms "agonist muscle" and "antagonist muscle" may be understood as the respective sets of muscles or muscle groups involved in the onset of the assessed cervical dystonia type and showing respectively unbalanced activity, with the agonist showing comparatively lower activity than the antagonist or vice versa.

The skilled person will appreciate that, depending on the type of cervical dystonia, the agonist muscle may show hypo-functional activity with respect to a healthy state, the antagonist muscle may show hyper-functional activity with respect to a healthy state, or both the agonist muscle and the antagonist muscle may each show hypo-functional activity and hyper-functional activity, respectively. Misfunction may lead to impaired coordination which can result in the above described abnormal head posture.

Impaired coordination may occur between agonists on one side of the neck and antagonists on the other side with respect to the sagittal or coronal plane; or it may occur between agonist and antagonist muscles on the same side of the neck with respect to the sagittal or coronal plane.

The inventors developed the system to counteract the deficit of coordination between the agonist and antagonist muscles by electrically stimulating one or both of the agonist and antagonist muscles with an electrode in functional proximity to the respective muscle. In this respect, functional proximity may be understood as a placement, such that the muscles and/or their innervating nerves may be stimulated (super-threshold stimulation) by the provision of electrical pulses via the electrode. The pulsed electrical stimulation signal is then adapted to reduce an imbalance in the activity of the agonist muscle and the antagonist muscle, and is preferably adapted to reduce an imbalance in the coordination of the activity of the agonist muscle and the antagonist muscle.

The first electrode may be placed adjacent to the respective muscle, over portions of the respective muscle or its innervating nerve, or maybe implanted partially in the respective muscle. For example, the first electrode may be an implantable electrode to be placed inside of or in direct proximity to the muscle or its innervating nerves, or may be a surface electrode to be placed on the skin of the neck of a patient adjacent to and/or over the respective muscle. The stimulator may be implanted with the first electrode or may be worn on the patient, e.g. the stimulator may adhere to the patient with the first electrode or may be attached to the skin or clothing of the patient distant from the first electrode.

The first electrode may be coupled to the stimulator through wired connections, which may be detachable or permanently fixed to the first electrode. The pulsed electrical stimulation signal generated by the stimulator may then be applied to the first electrode through the wires to locally affect an electrical potential in the respective muscle and/or associated nerves to selectively affect an activity of the agonist and/or antagonist muscle.

According to the present invention , the pulsed electrical stimulation signal is configured for reducing the imbalance in the activity of the agonist muscle and the antagonist muscle by one or both of increasing activity of the agonist muscle and reducing activity of the antagonist muscle.

The pulsed electrical stimulation signal may induce muscle activity in the agonist muscle and/or may reduce muscle activity in the antagonist muscle. For example, the pulsed electrical stimulation signal may be applied by the first electrode to trigger muscle activity in the agonist muscle, such as to reduce the imbalance in the activity of the agonist and the antagonist muscle. As another example, the pulsed electric stimulation may be applied by the first electrode to the antagonist muscle, such as to induce hyperpolarization in nerve bundles of the antagonist muscle to reduce an imbalance in the activity of the agonist and the antagonist muscle.

In preferred embodiments, the agonist muscle and/or the antagonist muscle are selected from the group of: musculus sternocleidomastoideus; musculus trapezius; musculus scaleni medius et posterior; musculus splenius capitis; musculus levator scapulae; musculus semispinalis capitis et cervicis; musculus splenii capitis et cervicis; musculus digastricus; musculus omohyoideus; platysma; musculus longus colli et capitis; musculus rhomboidei minor et major; musculus longisimus capitis et cervicis; musculus rectus lateralis; musculus rectus capitis posterior minor et major; musculus obliquus capitis superior et inferior; and musculus scalene complex.

For example, in torticollis, the musculus sternocleidomastoideus may show hypo-functional activity as an agonist muscle. Accordingly, the first electrode may be placed in functional proximity to the musculus sternocleidomastoideus to activate the musculus sternocleidomastoideus. In addition, in torticollis, the contralateral sternocleidomastoideus as an antagonist muscle may show hyperactive function, and the first electrode may be placed in functional proximity to the contralateral sternocleidomastoideus to reduce the activity of the muscle. Alternatively or additionally, in torticollis, the muscles splenius capitis and semispinalis capitis at the ipsilateral side with respect to the musculus sternocleidomastoideus may show hyper-functional activity and the first electrode may be placed in functional proximity to the contralateral sternocleidomastoideus to reduce the activity of the muscles.

The skilled person will appreciate that in cervical dystonia isolated muscles may show hyperactivity or hypoactivity, pairs of muscles may show respectively imbalanced hyperactivity and hypoactivity, or a plurality of muscles may show hyperactivity and/or hypoactivity. Hence, the terms "agonist muscle" and "antagonist muscle" should be construed as at least one of a group of agonist or antagonist muscles, respectively, depending on the type of cervical dystonia and the details of the condition. When a plurality of muscles show hyperactivity or hypoactivity as agonist muscles or antagonist muscles, the system may apply pulsed electrical stimulation signals to a subset or to each one of the agonist and/or antagonist muscles via a plurality of dedicated electrodes, and/or via a common electrode for a plurality of agonist or antagonist muscles.

Depending on the type of dystonia, e.g. whether the agonist shows the hypo-functional activity with respect to a healthy state, whether the antagonist muscle shows hyper-functional activity with respect to a healthy state, or whether both the agonist muscle and the antagonist muscle each show hypo-functional activity and hyper-functional activity, respectively, the stimulation strategy may be adapted to stimulate the agonist or the antagonist with a pulsed electrical stimulation signal, or to stimulate both the agonist and the antagonist with different pulsed electrical stimulation signals sequentially or simultaneously, e.g. through different electrode contacts.

In preferred embodiments, the first electrode is configured for being placed at the agonist muscle, wherein in embodiments of the claimed invention, the stimulator is configured to apply a biphasic electrical stimulation pulse to the first electrode suitable for increasing muscle activity of the agonist muscle.

The biphasic electrical stimulation pulse may be repeated in the pulsed electrical stimulation signal at predetermined time intervals, e.g. with a repetition frequency, and may feature successive positive and negative electrical excitation, or vice versa, to increase muscle activity of the agonist muscle.

The waveform of the biphasic electrical stimulation pulse may be balanced or unbalanced and may be symmetric or asymmetric depending on the patient and the muscle. For example, the biphasic electrical stimulation pulse may be symmetric to limit fatigue and/or minimize (skin) irritation risk. As an example, the biphasic electrical stimulation pulse may comprise of symmetric positive and negative pulses with balanced charge to address the agonist. However, the biphasic electrical stimulation pulse may also be asymmetric in embodiments, e.g. to target smaller muscle groups.

The biphasic electrical stimulation pulse may increase activity of the agonist to reduce an imbalance in the activity of the agonist and the antagonist muscle, e.g. when the agonist shows hypo-functional activity with respect to a healthy state.

In preferred embodiments, the pulsed electrical stimulation signal may have a frequency or an amplitude modulated signal envelope, such as a rectangular or sine signal envelope.

In preferred embodiments, the first electrode is configured for being placed at the antagonist muscle, and in embodiments of the claimed invention, the stimulator is configured to apply a triphasic electrical stimulation pulse to the first electrode suitable for reducing muscle activity of the antagonist muscle.

The triphasic electrical stimulation pulse may reduce activity of the hyper-functional antagonist, e.g. through hyperpolarization of nerves associated with the antagonist, to reduce an imbalance in the activity of the agonist and the antagonist muscle, e.g. when the antagonist shows hyper-functional activity with respect to a healthy state.

The triphasic electrical stimulation pulse should be charge balanced, e.g. by applying a triphasic electrical stimulation pulse with a first pulse of a first polarity, a second pulse having a second, opposite polarity, and a third pulse with the first polarity in sequence. The second pule may have a larger pulse width than the first pulse and the third pulse or may have a larger pulse amplitude than the first pulse and the third pulse, or a combination thereof, such that the integrated charge of the composite triphasic pulse is zero.

The triphasic electrical stimulation pulse may be applied with a monopolar electrode geometry, e.g. wherein the first electrode comprises a first contact in functional proximity of the antagonist muscle and a second grounding contact distant from the first electrode contact.

In preferred embodiments, the pulsed electrical stimulation signal has a triangular signal envelope.

The triangular signal envelope may improve an effect on the antagonist muscle activity to reduce an imbalance in the activity of the agonist and antagonist muscles. The pulsed electrical stimulation signal may comprise a plurality of pulse trains, wherein each of the pulse trains may have a triangular signal envelope. In other words, the pulse trains may have a varying amplitude, wherein the pulse amplitude is in particular ramped up from a low initial value to a high final value during each pulse train.

In preferred embodiments, the first electrode is placed or adapted to be placed on the agonist muscle and/or its innervating nerve, and the system comprises a second electrode. The second electrode is placed or adapted to be placed on the antagonist muscle and/or its innervating nerve(s). The pulsed electrical stimulation signal comprises a first pulsed electrical stimulation signal and a second pulsed electrical stimulation signal, wherein the stimulator is configured to apply the first pulsed electrical stimulation signal to the first electrode and to apply the second pulsed electrical stimulation signal to the second electrode to reduce the imbalance in the activity of the agonist muscle and the antagonist muscle.

Accordingly, the first pulsed electrical stimulation signal and the second pulsed electrical stimulation signal may be applied sequentially or synchronously to both increase activity of the agonist muscle and reduce activity of the antagonist muscle, e.g. when the agonist shows hypo-functional activity with respect to a healthy state and when the antagonist shows hyper-functional activity with respect to a healthy state.

For example, the stimulator may be configured to alternatingly apply the first pulsed electrical stimulation signal and the second pulsed electrical stimulation signal with overlapping or non-overlapping respective electrical pulses or pulse trains. The sequential application of the first pulsed electrical stimulation signal and the second pulsed electrical stimulation signal may improve a coordination of the activity of the agonist muscle and the antagonist muscle.

An alternating application of the first pulsed electrical stimulation signal and the second pulsed electrical stimulation signal may also reduce circuit and/or energy requirements of the stimulator, such as to enable reducing a weight and/or footprint of the stimulator.

The skilled person will appreciate that the stimulator may be a single unit connected to the first and the second electrode via respective wired connections, or may comprise a plurality of stimulator units for generating the pulsed electrical stimulation signal. For example, the first electrode and the second electrode may be coupled through wired connections to first and second stimulator units of the stimulator, respectively, for generating the first pulsed electrical stimulation signal and the second pulsed electrical stimulation signal. The respective stimulator units may operate individually and may be driven by an external control system communicating with the stimulator units through a wired or wireless connection.

The control system may comprise a single control unit or may comprise a plurality of control units which may be functionally connected. The control units may comprise a microcontroller, an ASIC, a PLA (CPLA), an FPGA, or other control device, including control devices operating based on software, hardware, firmware, or a combination thereof. The control devices can include an integrated memory, or communicate with an external memory, or both, and may further comprise interfaces for connecting to sensors, devices, appliances, integrated logic circuits, other controllers, or the like, wherein the interfaces may be configured to receive or send signals, such as electrical signals, optical signals, wireless signals, acoustic signals, or the like.

The stimulator units may comprise similar control devices, may comprise stimulation control units configured for generating the pulsed electrical stimulation signals, and may comprise interfaces for receiving manual user input or for connecting to the electrodes, sensors, other stimulator units or to the control system for receiving or sending signals, such as electrical signals, optical signals, wireless signals, acoustic signals, or the like.

For example, the first electrode and the second electrode may be associated with respective stimulator units, wherein the stimulator units may be configured to be worn by the patient, when the first electrode and the second electrode are placed on the agonist and antagonist muscle, respectively, and an external control system, such as a mobile phone, may set operating parameters and/or trigger operation of the stimulator units through a wireless connection to the stimulator units.

One of a plurality of stimulator units may assume a master configuration with respect to the other stimulator units, e.g. such that the control system may set operating parameters of the stimulator units through a main stimulator unit, while the other stimulator units may be configured and/or synchronized by the main stimulator unit. However, the operation of all the stimulator units may also be centrally controlled by an external control system.

A user may accordingly define operating parameters for each of the stimulator units through the control system, such as to jointly or individually control the application of the first pulsed electrical stimulation signal and the second pulsed electrical stimulation signal.

In preferred embodiments, the first and second pulsed electrical stimulation signals are different from each other, in particular with respect to at least one of the pulse shape, the amplitude, the signal envelope, the timing, and the duty cycle of the respective signal.

For example, the first pulsed electrical stimulation signal and the second pulsed electrical stimulation signal may both be configured to activate the agonist and the antagonist, but an increase of the activity induced by the first pulsed electrical stimulation signal and the second pulsed electrical stimulation signal may be different. For example, the first pulsed electrical stimulation signal may comprise pulses with a larger pulse width and/or amplitude than the second pulsed electrical stimulation signal and/or a greater number of pulses in a certain time window, to induce a larger relative activity increase in the agonist than in the antagonist.

In addition or alternatively, the first pulsed electrical stimulation signal may have a longer duty cycle than the second pulsed electrical stimulation signal. In some embodiments, the first pulsed electrical stimulation signal may comprise more active duty cycles than the second pulsed electrical stimulation signal. For example, the stimulator may be configured to apply a pulsed electrical stimulation signal to the first electrode in a first active cycle, to apply a pulsed electrical stimulation signal to the first electrode in a subsequent second active cycle, and to apply a pulsed electrical stimulation signal to the first electrode in a third active cycle, wherein a total activity increase of the antagonist may be smaller than a total activity increase of the agonist.

When both, the first pulsed electrical stimulation signal and the second pulsed electrical stimulation signal are adapted to increase an activity of the respective muscle to improve a deficit in coordination between the agonist muscle and the antagonist muscle, the first pulsed electrical stimulation signal and the second pulsed electrical stimulation signal may not be applied simultaneously, but sequentially, e.g. with non-overlapping pulses in time.

Preferably, the second pulsed electrical stimulation signal is adapted to reduce the activity of the antagonist muscle.

In preferred embodiments, the second pulsed electrical stimulation signal comprises triphasic electrical stimulation pulses adapted to reduce muscle activity of the antagonist muscle.

Accordingly, the activity of a hyperactive antagonist muscle may be reduced, e.g. to a physiologically correct/normal level, while a hypoactive agonist muscle may be activated, e.g. its activity may be increased to a clinically relevant activation level or a physiologically correct/normal level, to reduce an imbalance in the activity of the agonist muscle and the antagonist muscle.

The first electrode may feature a monopolar, bipolar, or multipolar (e.g. tripolar) electrode geometry adapted to couple the pulsed electrical stimulation signal to the agonist and/or antagonist muscle(s), e.g. as a rod electrode or a cuff electrode, such as a cuff electrode in functional proximity to an innervating nerve (e.g. around an innervating nerve).

In preferred embodiments, at least one of the first electrode and the second electrode comprises a first electrode contact and a second electrode contact, wherein the first electrode contact and the second electrode contact are connected to a respective first wire and a second wire, and wherein the stimulator is configured to apply a pulsed electrical stimulation signal of opposite polarity to the first electrode contact and the second electrode contact through the first wire and the second wire, respectively, and/or wherein the stimulator is configured to apply a pulsed electrical stimulation signal to the first electrode contact, while the second electrode contact is grounded.

When the second electrode contact is grounded, the respective electrode may be driven in a monopolar configuration, such as to apply hyperpolarizing electrical stimulation signals via the first electrode contact to the muscle, while the second electrode contact may act as a potential reference. The second electrode contact may have a larger contact area than the first electrode contact and may be placed or adapted to be placed in an area distinct from the respective agonist or antagonist muscle, such as to prevent direct stimulation of the respective agonist or antagonist muscle with the second electrode contact.

Preferably, the second electrode is in a monopolar configuration for reducing hyperactivity of the antagonist muscle through hyperpolarization of nerves of the antagonist muscle. However, the first electrode may also be provided in a monopolar configuration for increasing an activity of the agonist.

When pulsed electrical stimulation signals of opposite polarity are applied to the first electrode contact and the second electrode contact, the respective electrode may be driven in a bipolar or multipolar configuration. For example, the first electrode contact may be placed in functional proximity of a first muscle portion and the second electrode contact may be placed in functional proximity of a second muscle portion of an agonist or antagonist muscle to induce activity of the respective muscle by applying the pulsed electrical stimulation signals of opposite polarity to the first and second electrode contact. However, the first electrode or the second electrode may equally comprise at least three electrode contacts to increase or reduce activity in a plurality of agonist or antagonist muscles. The contact area of the first and second electrode contact may be similar in a bipolar or multipolar configuration.

In preferred embodiments, the first electrode contact and the second electrode contact are held in a relative geometric arrangement by an electrode body, in particular a common adhesive patch.

For example, the electrode may feature a common electrode body holding the first electrode contact and the second electrode contact in a pre-determined geometry, such as a pre-determined spacing. For example, a flexible patch may comprise first and second electrode contacts, wherein the adhesive patch may be adapted to be placed over a muscle on the neck of a patient, such that the adhesive patch substantially follows the surface of the neck at the location of the muscle and such that the first and second electrode contacts are spaced along said muscle.

The skilled person will appreciate that the electrode body may be flexible such as to adapt to the surface of the neck over the respective muscle(s) or to the shape of the moving muscle when the electrode is implanted. Accordingly, the pre-determined spacing should be construed as an at least partially variable spacing, such as a spacing within 30% of the pre-determined spacing. The flexible electrode body may accommodate a range of spacings between the electrode contacts, and the flexible electrode body or an elastic element in the electrode body may generate restoring forces towards the pre-determined spacing upon deformation, such that the electrode body may adjust to the anatomy of a patient within a predetermined adjustment range.

In some embodiments, the geometric arrangement is in particular adapted to the geometry of the agonist muscle and/or the antagonist muscle and/or their innervating nerves.

For example, a form of the common electrode body may be adapted to the geometry of the neck of a patient, such that the first and second electrode contacts are spaced along a predetermined muscle of the patient when the common electrode body is applied to the neck. In some embodiments, the common electrode body comprises a plurality of pairs of first and second electrode contacts, wherein the arrangement of the pairs of first and second electrode contacts on the common electrode body is adapted, such that different pairs of first and second electrode contacts are spaced along different predetermined muscles of the patient. The common electrode body may have the shape of a neck collar adjusted to the shape of the neck of the patient, or to a portion of the neck of the patient.

In preferred embodiments, at least one of the first electrode and the second electrode comprises a plurality of electrode contacts, wherein the stimulator is configured to select a subset of at least two electrodes among the plurality of electrodes for applying the pulsed electrical stimulation signal.

For example, the stimulator may be configured to selectively address a subset of at least two electrode contacts among the plurality of electrode contacts during a first duty cycle and may further be configured to subsequently address a different subset among the plurality of electrode contacts during a second duty cycle, such as to reduce/balance muscle fatigue arising from the application of the pulsed electrical stimulation signal over the area occupied by the plurality of electrode contacts, or to address different muscles.

In some embodiments, the plurality of electrode contacts occupy a contact area which is in functional proximity to a plurality of different muscles, and the subset of the at least two electrodes corresponds to a target muscle or muscle group below the contact area.

For example, the stimulator may apply the first or second pulsed electrical stimulation signal to different agonist or antagonist muscles of a group of agonist or antagonist muscles, respectively, in a sequential or simultaneous or synchronous manner. For example, the stimulator may apply the first pulsed electrical stimulation signal to a first agonist muscle at a first point in time and to a second agonist muscle at a second point in time, such as to sequentially apply the first pulsed electrical stimulation signal to different agonist muscles. Similarly, the stimulator may apply the second pulsed electrical stimulation signal to a first antagonist muscle at a first point in time and to a second antagonist muscle at a second point in time, such as to sequentially apply the second pulsed electrical stimulation signal to different antagonist muscles. In addition, the stimulator may also be configured to apply a different pulsed electrical stimulation signal to different agonist (or antagonist) muscles, e.g. may be configured to apply pulsed electrical stimulation signals having a different amplitude/pulse width/duty cycle to different agonist muscles, such as to adapt the respective pulsed electrical stimulation signals to an electrode geometry or anatomical position of the respective muscles.

The first electrode and the second electrode may also be provided in a common electrode body, for example as different subsets of a reconfigurable electrode contact array. For example a neck collar may feature a plurality of electrode contacts, and the plurality of electrode contacts may be arranged to correspond to the positions of different agonist and/or antagonists in cervical dystonia. The stimulator may address different subsets of the electrode contacts as a first and second electrode, such as to apply the first pulsed electrical stimulation signal and the second pulsed electrical stimulation signal with the common plurality of electrode contacts. In other words, the stimulator may implement first and second electrodes with different subsets of a plurality of electrode contacts of an electrode, e.g. a (reconfigurable) array of electrode contacts.

In some embodiments, an array of a plurality of electrode contacts is implanted in functional proximity of an agonist or antagonist muscle, and the stimulator may be configured to selectively address a subset of at least two electrodes among the plurality of electrodes, such as to maximize a response to the applied pulsed electrical stimulation signal after implantation.

In preferred embodiments, an amplitude of the pulsed electrical stimulation signal is smaller than 30 mA, in particular smaller than 15 mA.

For example, the amplitude of the pulsed electrical stimulation signal may be about 10 mA or lower.

In preferred embodiments, a pulse width of pulses in the pulsed electrical stimulation signal is smaller than 1 ms, in particular smaller than 0.5 ms, preferably smaller than 0.3 ms.

For example, pulses of opposite polarity a in a biphasic stimulation pulse waveform may each have a pulse width of 0.2 ms, or a triphasic stimulation pulse waveform may feature two pulses having pulse width of 0.1 ms and one center pulse of opposite polarity with a pulse width of 0.2 ms.

In preferred embodiments, the repetition frequency of pulses in the pulsed electrical stimulation signal is smaller than 120 Hz, and in particular 60 Hz or lower. For example, the pulses in the pulsed electrical stimulation signal may be repeated at a repetition frequency of about 60 Hz or may be about 40 Hz or lower, such as 30 Hz or 20 Hz, e.g. to strengthen the agonist muscle during a training session adapted to reduce the imbalance in the activity of an agonist muscle and an antagonist muscle.

In preferred embodiments, the stimulator is configured to apply the pulsed electrical stimulation signal with a duty cycle comprising a high intensity cycle and a low intensity cycle, wherein the stimulator switches between the high intensity cycle and the low intensity cycle in regular time intervals, wherein a duration of the high intensity cycle and the low intensity cycle is in particular smaller than 10 ms, and wherein a duration of the high intensity cycle is in particular larger than a duration of the low intensity cycle.

For example, the pulsed electrical stimulation signal may be switched on for a duration of about five seconds and may be switched off for a duration of about five seconds or may be switched off for two seconds, such as to reduce discomfort of the patient.

In preferred embodiments, the stimulator is configured to apply the pulsed electrical stimulation signal for a duration of at least 10 min and/or for at most 60 min, in particular for a duration between 20 min and 40 min.

The cervical dystonia may be treated with the system with a training program of stimulating the activity of one or both of the agonist and the antagonist muscle by applying the pulsed electrical stimulation signal through the first electrode and/or second electrode in regular intervals, such as a predetermined number of times per day or per week, e.g. three times per day or per week. Each training session may have a duration of between 10 minutes and 30 minutes to reduce an imbalance in the activity of the agonist muscle and the antagonist muscle.

Depending on the progression of the cervical dystonia, the stimulator may be configured to apply deposit electrical stimulation signal adapted to increase a strength of the agonist muscle, or maybe adapted to reduce an imbalance in the coordination of the agonist and the antagonist muscle.

In preferred embodiments, the first electrode is self-adhesive to the skin of a patient, and in particular repeatedly attachable to the skin of the patient.

Accordingly, the first electrode may be attached to the skin of the neck for a training session at may be removed following the training session. The first electrode may be attached to the skin of the neck by the patient, e.g. after a detailed instruction by a specialist.

In some embodiments, a first stimulator unit is fixedly coupled to the first electrode and is configured to adhere to the skin of the patient with the first electrode. For example, the first electrode contact and the second electrode contact may be held in a fixed geometric arrangement by an adhesive patch to be placed over an agonist or antagonist muscle of the neck, wherein the adhesive patch may hold a stimulator unit of the stimulator. The second electrode may be coupled to the same stimulator unit through wired connections or may be associated with a second stimulator unit fixedly coupled to the second electrode and equally configured to adhere to the skin of the patient with the second electrode.

Accordingly, the first electrode and the second electrodes as well as associated stimulator units may be provided as a modular system which may be configured to adhere to the skin of the neck of the patient for regular training sessions.

In some embodiments, the stimulator may be configured to apply the pulsed electrical stimulation signal at regular time intervals and/or in response to a sensor signal, such as to reduce an acute hypoactivity the of an agonist and/or an acute hyperactivity of an antagonist.

For example, the stimulator may be coupled to a sensor or may be triggered to apply the pulsed electrical stimulation signal based on measurement readings of a sensor indicating a prolonged hyperactivity of an antagonist and/or based on detection of the head assuming a an abnormal posture for a predetermined duration. For example, the system may automatically detect an abnormal posture due to hyperactivity of an antagonist or hypoactivity of an agonist with the sensor and drive the stimulator to treat acute symptoms of the cervical dystonia.

In preferred embodiments, the system further comprises a sensor to detect an activity of the agonist muscle and/or the antagonist muscle.

In preferred embodiments, the first electrode is an implantable electrode, wherein the system comprises an implant which includes the stimulator.

Implanting the first electrode may reduce a risk of user error during surface application of the first electrode and may also enable prolonged application of the pulsed electrical stimulation signal without requiring (visible) surface electrodes present on the neck of the patient. For example, when a training using the pulsed electrical stimulation signal is or becomes ineffective in a patient, the patient may be a provided with the implantable electrode to constantly and/or dynamically reduce the imbalance in the activity of the agonist muscle and the antagonist muscle with the system.

In some embodiments, the system further comprises a non-implantable outside component configured to be worn on the body of a patient and configured to couple to the implant.

For example, the implant may be provided with an induction coil to receive energy from the outside component for generating the pulsed electrical stimulation signal. The outside component may connect to the implant through a wireless interface, such as to change operating parameters of the stimulator or stimulator unit implanted with the first electrode. However, in some embodiments, the implant may be directly connected to an external control system, such as a mobile terminal, to receive operating parameters for the stimulator or stimulator unit.

A second electrode may equally be configured as an implantable electrode and may be coupled to the implant, or may be provided with a second implant with an independent stimulator unit, such as for locally generating pulsed electrical stimulation signals for each of the first and second electrodes.

In preferred embodiments, the stimulator can be switched to a fitting mode, in which the stimulator is configured to vary at least one of the pulse amplitude, the pulse width, and the pulse frequency of the pulsed electrical stimulation signal.

In preferred embodiments, in the fitting mode, the system receives sensor readings from a sensor monitoring the activity the agonist muscle and/or the antagonist muscle in response to the electrical stimulation signal.

In a second aspect, the disclosure relates to a non-claimed computer-implemented method for fitting a system for treating cervical dystonia based on a series of measurements of a sensor monitoring the activity an agonist muscle and/or an antagonist muscle. The method comprises varying at least one of the pulse amplitude, the pulse width, and the pulse frequency of the pulsed electrical stimulation signal, and receiving sensor readings from the sensor monitoring the activity of the agonist muscle and/or the antagonist muscle in response to the pulsed electrical stimulation signal. The method further comprises determining whether the sensor readings overcome a pre-determined activity threshold, and determining a value of the at least one of the pulse amplitude, the pulse width, and the pulse frequency of the pulsed electrical stimulation signal for reducing an imbalance in the activity of an agonist muscle and an antagonist muscle.

In a third aspect, the disclosure relates to a non-claimed computer program comprising machine readable instructions, which when the computer program is executed by a processing unit cause the processing unit to implement the method according to the second aspect and/or to implement and/or to control a system according to the first aspect.

In a fourth aspect, the disclosure relates to a non-claimed use of the system according to the first aspect for treating cervical dystonia.

In a fifth aspect, the disclosure relates to a non-claimed method of treating cervical dystonia using the system according to the first aspect. The method comprises placing a first electrode in functional proximity to at least one of an agonist muscle and an antagonist muscle and/or their innervating nerves. The method further comprises driving a stimulator coupled to the first electrode for applying a pulsed electrical stimulation signal to the first electrode, wherein the pulsed electrical stimulation signal is configured for selectively increasing or reducing the activity of the at least one of the agonist muscle and the antagonist muscle, wherein the pulsed electrical stimulation signal is adapted to reduce an imbalance in the activity of the agonist muscle and the antagonist muscle.

The method may comprise diagnosing symptoms of cervical dystonia in a patient which may comprise determining whether the chin is twisted towards a shoulder so that the head rotates sideways; or whether the head tips forward; or whether the head is tilted backward; or whether the head tilts toward one side; or whether the head is shifted on the shoulders in a forward (anterior sagittal shift) or backward (posterior sagittal shift) direction.

The method may further comprise identifying at least some of the muscles involved causing these symptoms. Determining muscles involved may include to visual inspection, EMG measurement in proximity to the muscle, and/or electroneuronography (ENog) examinations. The method may then comprise identifying muscles showing hypo-functional activity as agonists and muscles showing hyper-functional activity as antagonists.

In some embodiments, the method comprises placing a first electrode in functional proximity to an agonist muscle and/or its innervating nerve and placing a second electrode and functional proximity to an antagonist muscle and/or its innervating nerve.

The method may then comprise triggering the stimulator to generate the pulsed electrical stimulation signal to at least one of the first electrode and the second electrode.

### DETAILED DESCRIPTION OF EMBODIMENTS

The features and numerous advantages of the system and fitting method according to the present invention will best be understood from a detailed description of preferred embodiments with reference to the accompanying drawings, in which:
- Fig. 1: shows a schematic system for treating cervical dystonia illustrated with an example of latero-/torticollis;
- Fig. 2A, B: illustrate exemplary biphasic and triphasic pulses of a pulsed electrical stimulation signal for activating and deactivating a target muscle;
- Fig. 2C-E: illustrate exemplary portions of a pulsed electrical stimulation signal;
- Fig. 3: illustrates another example of a schematic system for treating cervical dystonia illustrated with an example of latero-/torticollis;
- Fig. 4: illustrates a schematic view of a stimulation system for treating cervical dystonia according to an example;
- Fig. 5A, B: illustrate an example of a stimulator comprising a plurality of stimulator units with respective associated electrodes;
- Fig. 6: illustrates a schematic stimulation system for treating cervical dystonia according to another example;
- Fig. 7A, 7B: illustrates an example of a system for treating cervical dystonia with first and second stimulation units;
- Fig. 7C, 7D: illustrates another example of a system for treating cervical dystonia;
- Fig. 8A, 8B: illustrate bottom and side views of an implantable electrode according to an example;
- Fig. 8C: illustrates another example of an implantable electrode comprising an array of electrode contacts;
- Fig. 8D: illustrates an example of an implant with an integrated stimulation unit; and
- Fig. 9: shows a schematic flowchart of a treatment method for treating cervical dystonia according to an example.

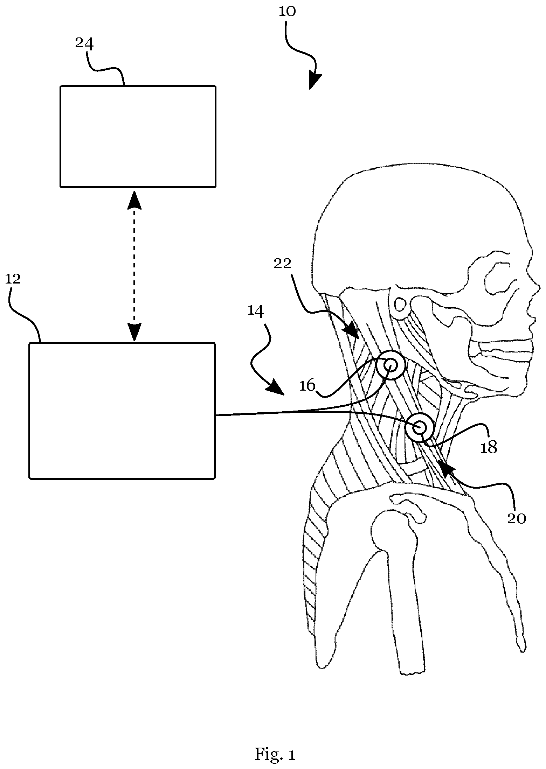

Fig. 1 shows a schematic system 10 for treating cervical dystonia illustrated with an example of latero-/torticollis. The system 10 comprises a stimulator 12 and a first electrode 14. The first electrode 14 comprises a first electrode contact 16 and a second electrode contact 18 with respective wired connections to the stimulator 12. The first electrode contact 16 and a second electrode contact 18 of the first electrode 14 are placed over the musculus sternocleidomastoid acting as an agonist muscle 20 in latero-/torticollis, e.g. with respect to the ipsilateral musculus splenius capitis acting as an antagonist muscle 22.

The stimulator 12 is coupled to the first electrode 14 through the wired connections and is configured to apply a pulsed electrical stimulation signal to the first electrode 14, e.g. by applying a pulsed potential difference between the first electrode contact 16 and the second electrode contact 18 of the first electrode, such that the local electrical potential in the muscle may be dynamically altered. The pulsed electrical stimulation signal can be configured for selectively increasing the activity of the agonist muscle 20 by adjusting a local polarization of a nerve of the agonist muscle 20 for increasing activity in the agonist muscle 20.

The inventors believe that in cervical dystonia arises due to a deficit in coordination of a hypo-functional agonist and hyper-functional antagonist is impaired, and the pulsed electrical stimulation signal generated by the stimulator 12 should therefore be adapted to reduce an imbalance in the activity of the agonist muscle 20 and the antagonist muscle 22, e.g. to restore the correct posture of the head, neck, and upper chest in patients suffering from cervical dystonia.

Patients may use the stimulation system 10 for therapeutic purposes twice to three times per day for about 30 min per session. After some therapeutic sessions, in many cases patients may have relief from their symptoms.

Although the first electrode 14 is shown to be placed in functional proximity to the agonist muscle 20 in Fig. 1, the first electrode 14 may also be placed on the antagonist muscle 22, and the pulsed electrical stimulation signal may be configured for selectively reducing the activity of the antagonist muscle 22, while the effectiveness of applying the pulsed electrical stimulation signal to the agonist muscle 20 and/or the antagonist may also depend on the condition of the patient and/or the muscles involved.

The stimulator 12 may be worn by the patient, e.g. in a pocket or attached to a belt, or may be attached to the body of the patient with the first electrode 14. The stimulator 12 may comprise a power source, e.g. a battery, or may be coupled to an external power source to power a signal generator for generating the pulsed electrical stimulation signal.

The operation of the stimulator 14 may be turned on with an activating switch and/or may be controlled with an optional control system 24, which may in wired or wireless connection to the stimulator 12. For example, the stimulator 12 may be initially programmed to generate a pre-determined pulsed electrical stimulation signal based on a set of operation parameters, such as pulse shape, frequency, amplitude, duty cycle, etc., and may be switched on with an activating switch to generate the pulsed electrical stimulation signal, e.g. for a pre-determined time period, such as 20 min or 30 min.

The operation of the stimulator 12 may be dynamically controlled with the control system 24, e.g. through a wireless connection to a terminal of the control system 24, such as a smartphone. For example, an operator may select operation parameters of the stimulator 12 on the terminal and may initiate the generation of the pulsed electrical stimulation signal through the terminal.

The signal generator 12 may generate a series of stimulation pulses, e.g. a pulse train with pulses regularly spaced in time, based on the operation parameters to induce a change in the activity of the targeted muscle 20, 22.

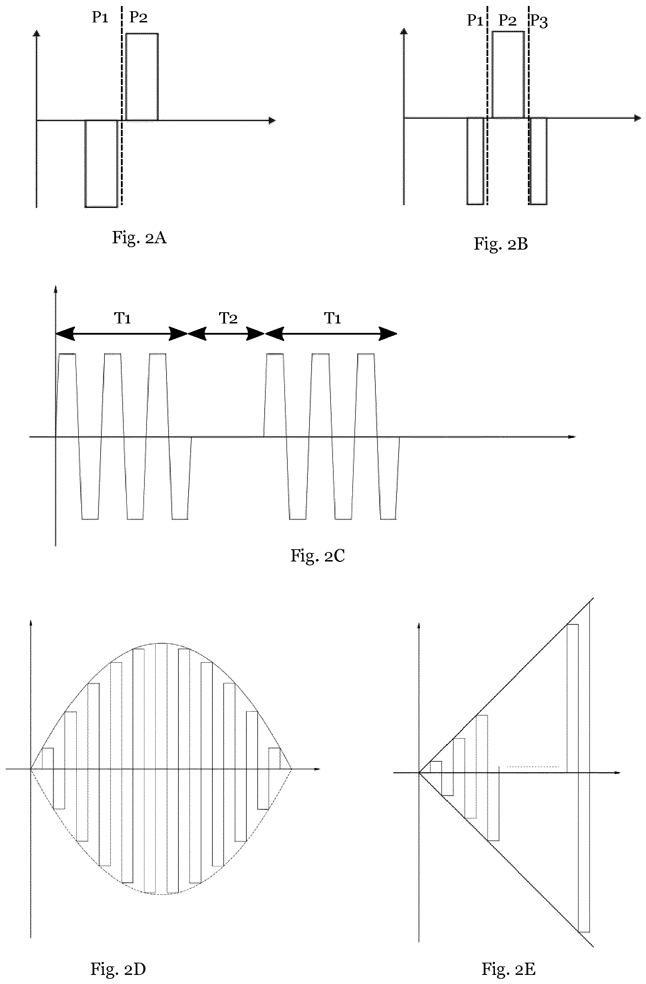

Fig. 2A and 2B illustrate exemplary biphasic and triphasic pulses of a pulsed electrical stimulation signal for activating and deactivating a target muscle 20, 22, while Fig. 2C-E illustrate exemplary portions of a pulsed electrical stimulation signal. In each of the figures, the vertical axis indicates the signal amplitude in arbitrary units, e.g. as a current or a voltage, while the signal is presented as a function of time (progressing along the horizontal axis).

In Fig. 2A, a biphasic pulse is illustrated according to an example, wherein the biphasic pulse comprises a first pulse segment P1 and a second pulse segment P2, the first and second pulse segments P1, P2 having opposite polarity. In the illustrated example, the first and second pulse segments P1, P2 have equal amplitude and equal pulse width, e.g. the same current amplitude of 10 mA at respective pulse widths of 0.2 ms. The amplitude may be a pulse amplitude for overcoming a patient specific stimulation threshold, e.g. affected by the tissue composition (local body fat percentage, scaring, etc.) and may be affected by the patient's treatment history, e.g. in the case of previous botulinum toxin injections.

It is noted that each of the pulse segments P1, P2 in fig. 2A is of rectangular shape. However, in general arbitrary wave forms may be employed in embodiments, e.g. the biphasic pulse may feature a sine wave form or may feature triangular wave forms for each pulse segment, and the pulse segments need not be symmetric.

The biphasic pulse may be charge balanced and symmetrical as illustrated in Fig. 2A, e.g. to increase the activity of a large agonist muscle group, or may be asymmetrical and unbalanced in embodiments.

Biphasic pulses may be preferred for increasing the activity of a target muscle. However, multiphasic pulses, e.g. triphasic pulses, Russian stimulation, etc., or monophasic pulses may equally be employed in embodiments to increase the activity of an agonist muscle (group).

In Fig. 2B, a triphasic pulse is illustrated according to an example, wherein the triphasic pulse comprises a first pulse segment P1 a second pulse segment P2 and a third pulse segment P3. The first and third pulse segments P1, P3 have the same polarity and amplitude, while the second pulse segment P2 between the first and third pulse segments P1, P3 has inverse polarity. All of the first through third pulse segments P1-P3 share the same amplitude. However, the pulse widths of the first and third pulse segments P1, P3 are different from the pulse width of the second pulse segment P2, in particular correspond to half the pulse width of the second pulse segment P2, in order to balance a charge of the triphasic pulse during stimulation.

The triphasic pulse may be employed for selectively reducing an activity of an antagonist muscle 22, e.g. by hyperpolarizing a nerve of the antagonist muscle 22 in functional proximity of the first electrode 14. The triphasic pulse may be applied with a monopolar electrode configuration, e.g. to isolate a hyperpolarizing effect of the triphasic pulse.

Independent of the pulse shape, the pulses may be applied as part of a pulse train comprising a sequence of a plurality of pulses for increasing activity of the agonist muscle 20 and/or for reducing activity of the antagonist muscle 22.

Fig. 2C illustrates a pulsed electrical stimulation signal with biphasic pulses according to an example. The pulsed electrical stimulation signal comprises pulse trains of a plurality of biphasic pulses in succession, wherein two identical pulse trains are applied during active cycles with a first duration T1 and the pulse trains are separated by an idle cycle with a second duration T2. The idle cycle may be an "off" period in which no signal is applied to the first electrode 14 or may be a reduced activity period, wherein an amplitude of the pulsed electrical stimulation signal is reduced.

As an example, the pulsed electrical stimulation signal may be applied during an active "ON" cycle, e.g. with a duration T1 of about 5s, and may be not applied during an idle "OFF" cycle e.g. with a duration T2 of 2s, with the active "ON" cycle and the idle "OFF" cycle defining a duty cycle for the pulsed electrical stimulation signal.

The pulse trains are applied in Fig. 2C with a rectangular envelope, i.e. the amplitude of the pulses in the pulse train is substantially constant. For example, the pulses in the pulse train may have a pulse amplitude of about 10 mA (or about 10V when voltage regulated), and may be applied with a repetition frequency of about 60Hz or about 40 Hz.

However, the amplitude of the pulses may also be varied during a pulse train in the active cycle, e.g. the signal envelope may be varied according to a (partial) sine waveform as illustrated in Fig. 2D, or according to a triangular waveform as illustrated in Fig. 2E. Accordingly, the maximum amplitude of the pulse train may be about 10 mA, e.g. in the middle of the pulse train and may be lower at the beginning (and the end) of the pulse train.

In some embodiments, the pulsed electrical stimulation signal may be applied to the agonist muscle 20 with a signal envelope according to a square waveform or a sine waveform, and an pulsed electrical stimulation signal may be applied to the antagonist muscle 22 with a signal envelope according to a triangular waveform, such as to reduce the imbalance in the activity of the agonist muscle and the antagonist muscle by one or both of reducing activity of the agonist muscle 20 and reducing activity of the antagonist muscle 22.

However, first and second pulsed electrical stimulation signals with the same signal envelope may also be applied to the agonist and antagonist muscles 20, 22, or a first pulsed electrical stimulation signal having a triangular signal envelope may be applied to the agonist muscle 20, while a second pulsed electrical stimulation signal with a square or sine signal envelope may be applied to the antagonist muscle 22 in embodiments.

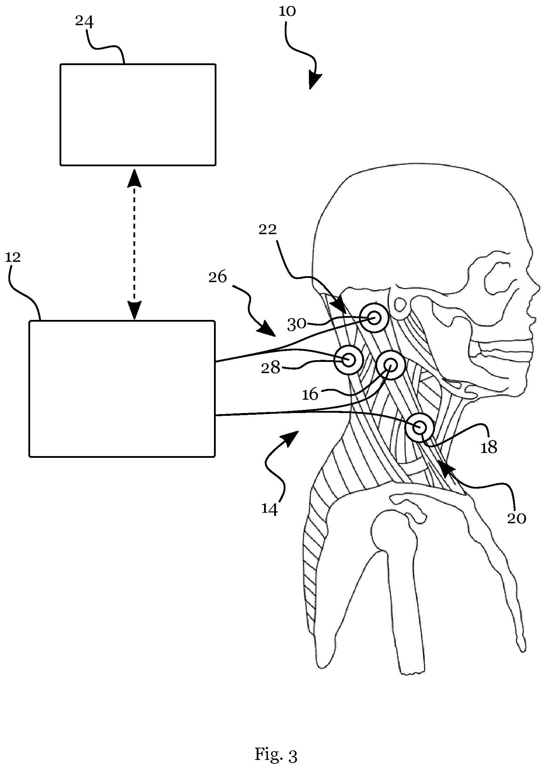

Fig. 3 illustrates another example of a schematic system 10 for treating cervical dystonia illustrated with an example of latero-/torticollis. The system 10 comprises a stimulator 12, a first electrode 14 and a second electrode 26. The first electrode 14 and the second electrode 26 each comprise a first electrode contact 16, 28 and a second electrode contact 18, 30 with respective wired connections to the stimulator 12.

The first electrode contact 16 and a second electrode contact 18 of the first electrode 14 are placed over the musculus sternocleidomastoid acting as an agonist muscle 20 in latero-/torticollis. The first electrode contact 28 and a second electrode contact 30 of the second electrode 26 are placed over the ipsilateral musculus splenius capitis acting as an antagonist muscle 22 to the musculus sternocleidomastoid, in the illustrative example of latero-/torticollis.

The stimulator 12 may then be configured to apply a first pulsed electrical stimulation signal to the first electrode 14 and to apply a second pulsed electrical stimulation signal to the second electrode 26 to reduce the imbalance in the activity of the agonist muscle 20 and the antagonist muscle 22. Preferably, the first and second pulsed electrical stimulation signal are different from each other, such as to selectively increase the activity of the agonist muscle 20 with the first pulsed electrical stimulation signal applied to the first electrode 14 and to selectively reduce the activity of the antagonist muscle 22 with the second pulsed electrical stimulation signal applied to the second electrode 26.

For example, the first pulsed electrical stimulation signal and the second pulsed electrical stimulation signal may comprise pulses with different pulse shape, e.g. the first pulsed electrical stimulation signal may comprise a series of monophasic or biphasic pulses, and the second pulsed electrical stimulation signal may comprise a series of triphasic pulses.

In addition or alternatively, the second pulsed electrical stimulation signal may be applied with a different signal envelope, e.g. a triangular signal envelope as illustrated in Fig. 2E, or and may be applied with a different pulse amplitude, pulse width, or duty cycle.

For example, the pulse shape of the first pulsed electrical stimulation signal and of the second pulsed electrical stimulation signal may both be adapted to increase the activity of the respective agonist/antagonist muscle 20, 22, but an amplitude, pulse width or duty cycle of the second pulsed electrical stimulation signal may be different from the first pulsed electrical stimulation signal, such as to reduce a relative activity difference between the agonist muscle 20 and the antagonist muscle 22.

As another example, the stimulator 12 may generate the first pulsed electrical stimulation signal and the second pulsed electrical stimulation signal, such that the first pulsed electrical stimulation signal comprises a higher number of pulses or active cycles than the second pulsed electrical stimulation signal, such as to reduce a relative activity difference between the agonist muscle 20 and the antagonist muscle 22 during a training session.

Accordingly, the imbalance in the activity of the agonist muscle 20 and the antagonist muscle 22 may be treated by electrically stimulating the respective muscles with different stimuli sequentially or simultaneously via the first electrode 14 and the second electrode 26, such as to restore the correct posture of the head, neck, and upper chest in patients suffering from cervical dystonia.

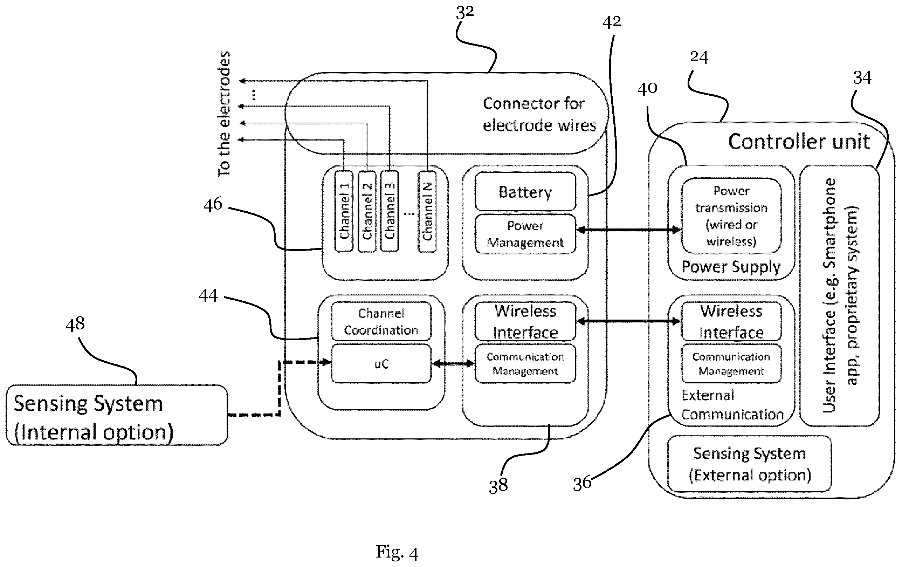

Fig. 4 illustrates a schematic view of a system 10 for treating cervical dystonia according to an example. The system 10 comprises a controller unit 24, and a stimulator unit 32 coupled to a plurality of electrodes 14, 26 (not explicitly shown). The controller unit 24 may be a smartphone with control software for interacting with the stimulator unit 32. For example, a user may input operation parameters of the stimulator unit 32 through a user interface 34, such as to trigger the application of a pulsed electrical stimulation signal to the electrodes 14, 26.

The control unit 24 comprises a communication interface 36 to communicate with a corresponding communication interface 38 of the stimulator unit 32, such as to transmit control signals from the control unit 24 to the stimulator unit 32 and/or to receive operation status information from the stimulator unit 32, e.g. through a wireless communication path.

In some embodiments, the controller unit 24 comprises a power interface 40 to transmit electrical power to a power source 42 of the stimulator unit 32. For example, instead of being applied to the surface of the patient, the stimulator unit 32 may be implanted in the patient, e.g. on the sternum of the patient, and the controller unit 24 may be configured to inductively couple to the power source 42 of the stimulator unit 32 through the power interface 40, e.g. to charge a battery of the stimulator unit 32 and/or to power the stimulator unit 32 during operation.

The wireless interface 38 of the stimulator unit 32 may be coupled to a control device 44 of the stimulator unit 32, such as control device 44 comprising a microcontroller, an ASIC, a PLA (CPLA), an FPGA, or a combination thereof, to receive operation parameters at the control device 44. The control device 44 may be configured to generate the pulsed electrical stimulation signal by changing electrical potential at interfaces of the control device 44, or may be configured to drive and/or control operation of an internal pulse generator for generating the pulsed electrical stimulation signal(s) and/or to coordinate an application of the pulsed electrical stimulation signal(s) to at least one of the electrodes 14, 26 connected to the stimulator unit 32 through a connector interface 46.

In some embodiments, the stimulator unit 32 may be coupled to an internal sensor 48b, such as an EMG electrode in proximity to the muscle, and/or electroneuronography (ENog) device, such as to measure an activity of an agonist muscle 20 and/or an antagonist muscle 22, prior to or during application of the pulsed electrical stimulation signal signal(s) to at least one of the electrodes 14, 26 connected to the stimulator unit 32. In some examples, an external sensor 48a may be temporarily connected to the stimulator unit 32 or the control device 44 through a wired or wireless interface (not shown) to fit the operation parameters of the stimulator unit 32 today patient, e.g. to adjust an amplitude or pulse width to the anatomic specifics of the patient (e.g. the thickness of a fat layer beneath the skin). In some embodiments, the sensor 48a, 48b may be attached to an adhesive body of the stimulator unit 32, such as to adjust operation parameters of the stimulator unit 32 prior to a training session based on a measurement of the activity of the agonist muscle 20 and/or the antagonist muscle 22 in response to a variation of the operation parameters of the stimulator unit 32, before a treatment is performed.

The stimulator unit 32 may be a single unit, e.g. to be worn on the body of the patient and to be connected to the first electrode 14 and/or the second electrode 26, or may be part of a stimulator 12 comprising a plurality of stimulator units 32, e.g. first and second stimulator units 32 for applying the pulsed electrical stimulation signal to the first electrode 14 and the second electrode 26, respectively.

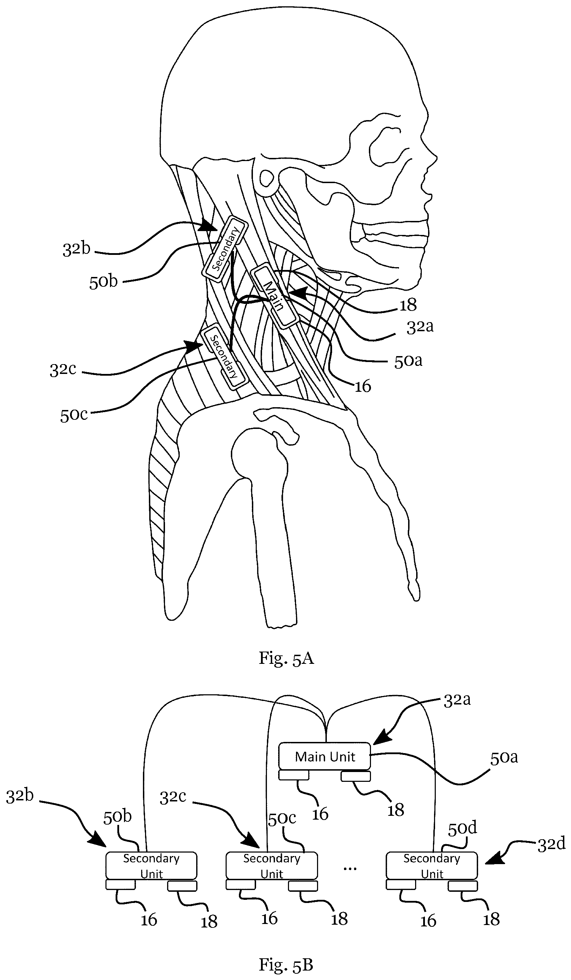

Fig. 5A, 5B illustrate an example of a stimulator 12 comprising a plurality of stimulator units 32a-d with respective associated electrodes 14, 26.

In Fig. 5A a main stimulator unit 32a is arranged over the musculus sternocleidomastoid acting as an agonist muscle 20 in latero-/torticollis, and comprises a first and a second electrode contact 16, 18 spaced along an electrode body 50a holding the main stimulator unit 32a. A first secondary stimulator unit 32b is arranged over the ipsilateral musculus splenius capitis acting as an antagonist muscle 22 with respect to the musculus sternocleidomastoid in latero-/torticollis. Further, for illustration purposes, a second secondary stimulator unit 32c is arranged over the musculus trapezius, e.g. to treat a latero-/torti-/ante or retrocaput condition.

Each of the first and second secondary stimulator units 32b, 32c comprises an electrode body 50b, 50c with respective first and second electrode contacts 16, 18 to adhere to a neck of a patient and to apply respective pulsed electrical stimulation signals to respective muscles in functional proximity of the electrode body 50b, 50c.

In other words, each of the stimulator units 32a-d may be attached to an electrode body 50a-d defining a geometry of electrode contacts 16, 18 of an associated electrode 14, 26, such that the stimulator units 32a-d may adhere to the skin of the neck of a patient together with an associated electrode 14, 26 through the electrode body 50a-d as a compact treatment unit.

As also shown in Fig. 5B, the first and second secondary stimulator units 32b, 32c, may be connected through wired connections 52 to the main stimulator unit 32a, such as to receive power, control signals, or the respective pulsed electric stimulation signal from the main stimulator unit 32a. However, it is noted that the wired connections are for illustration purposes only and the stimulator units 32a-d may also be connected through wireless communication paths in embodiments, without wired connections between the stimulation units 32a-c.

For example, each of the first and second secondary stimulator units 32b, 32c may comprise an internal signal generator to generate respective pulsed electric stimulation signals to be applied to the associated electrode contacts 16, 18, and may comprise internal power sources for driving the internal signal generator.

The main stimulator unit 32a may be configured to synchronize operation of the plurality of stimulator units 32a-d and/or to communicate with a control system 24 (not shown) in order to receive operation parameters for the pulsed electrical stimulation signal, and the main stimulator unit 32a may be configured to control the application of the pulsed electrical stimulation signal at any one of the stimulator units 32a-d.

In some embodiments, the main stimulator unit 32a comprises different components than the first and second secondary stimulator units 32b, 32c. However, the structure of the main stimulator unit 32a and the secondary stimulator units 32b-c may also be the same, and the main stimulator unit 32a may only be a designated main stimulator unit 32a, e.g. to synchronize operation of the plurality of stimulator units 32a-d in response to control signals of a control system 24 to the designated main stimulator unit 32a.

Moreover, in some embodiments, the control system 24 individually controls each of the stimulator units 32a-d without designating main or secondary stimulator units 32a-d.

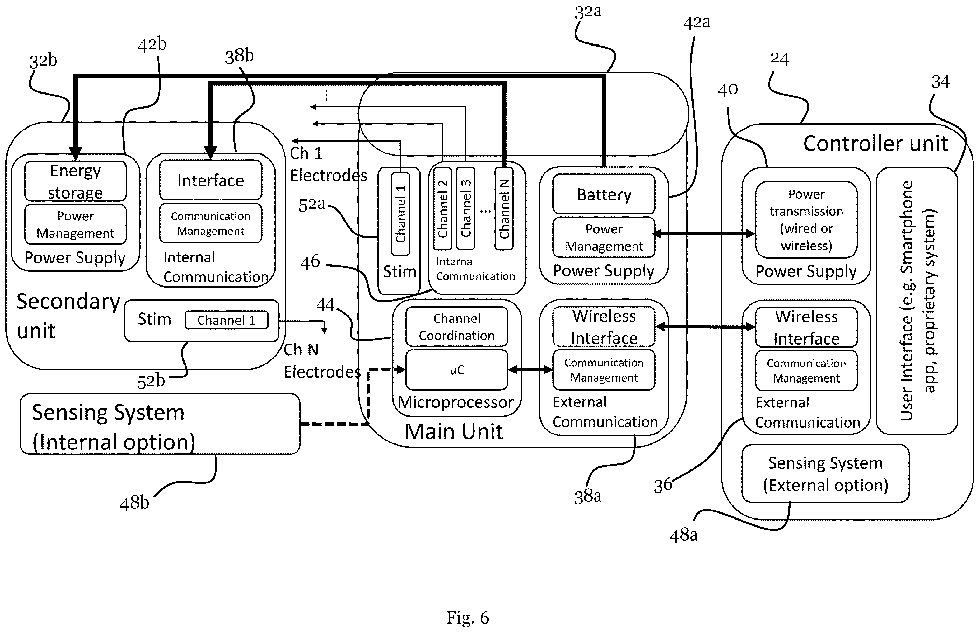

Fig. 6 illustrates a schematic stimulation system 10 for treating cervical dystonia according to another example with a dedicated main stimulation unit 32a. The system 10 comprises a controller unit 24, the main stimulator unit 32a and a secondary stimulator unit 32b, wherein the structure of the controller unit 24 is similar to the structure of the controller unit 24 illustrated in Fig. 4.

The controller unit 24 is configured to communicate with a communication interface 38a of the main stimulator unit 32a through a communication interface 36, e.g. to transmit control signals regarding the operation parameters of a pulsed electrical stimulation signal from the control unit 24 to the main stimulator unit 32a.

The main stimulator unit 32a comprises a power source 42a, a control device 44, a connector interface 46, and a stimulation control unit 52a. The control device 44 may be powered by the power source 42a and may communicate through the communication interface 38a of the main stimulation unit 32a, such as to receive and process control signals from the control unit 24. The control device 44 may determine operation parameters for the pulsed electrical stimulation signal(s) from the control signals and may control the stimulation control unit 52 and/or the connector interface 46 based on the operation parameters.

The stimulation control unit 52a may comprise a signal generator circuit for generating a first electrical stimulation signal associated with a set of operation parameters, such as a pulse width, a pulse amplitude, a pulse repetition frequency, and/or a duty cycle, to be applied to a first electrode 14 through an electrode interface (depicted as "channel 1").

The connector interface 46 may enable communication of the control device 44 with a communication interface 38b of the secondary stimulation unit 32b, such as to transmit control instructions from the main stimulation unit 32a to the secondary stimulation unit 32b, e.g. control instructions comprising operation parameters for generating a second pulsed electrical stimulation signal in the secondary stimulation unit 32b.

As illustrated in Fig. 6, the secondary stimulation unit 32b may comprise an internal stimulation control unit 52b, which may comprise a signal generator circuit, and a power source 42b to provide power to the stimulation control unit 52b and/or the communication interface 38b. The stimulation control unit 52b may be configured to generate a second pulsed electrical stimulation signal to be applied to a second electrode 26, e.g. placed on an antagonist muscle 22.

In operation, the main and secondary stimulation units 32a, 32b may be placed in functional proximity of different muscles, a user may input stimulation parameters through a user interface 34 of the control system 24.

The operation parameters may be received at the main stimulator unit 32a and may be processed by an internal control device 44 of the main stimulation unit 32a to generate control instructions for signal generation circuits of the main stimulation unit 32a, e.g. in the internal stimulation control unit 52a, and/or of the secondary stimulation unit 32b, which may be transmitted through the connector interface 46 to the communication interface 38b of the secondary stimulation unit 32b.

The main stimulator unit 32 and the secondary stimulation unit 32b may then be configured to generate first and second electrical stimulation signals, respectively, to be applied to first and second electrodes 14, 26 connected to the respective stimulation unit 32a, 32b.

Accordingly, the first and second electrical stimulation signals may be applied to the first and second electrodes 14, 26 in a synchronized manner, such as for reducing the imbalance in the activity of the agonist muscle 20 and the antagonist muscle 22, e.g. by increasing activity of the agonist muscle 20 and reducing activity of the antagonist muscle 20.

Preferably, a shape of an electrode body 50a-d carrying electrode contacts 16, 18, 28, 30 of the first and/or a second electrodes 14, 26 is adapted to a geometry of a target muscle or muscle group 20, 22. For example, the electrode body 50a-d may be adjusted to or may be adapted to be adjustable to the surface of the neck of a patient in functional proximity of a target muscle or muscle group 20, 22.

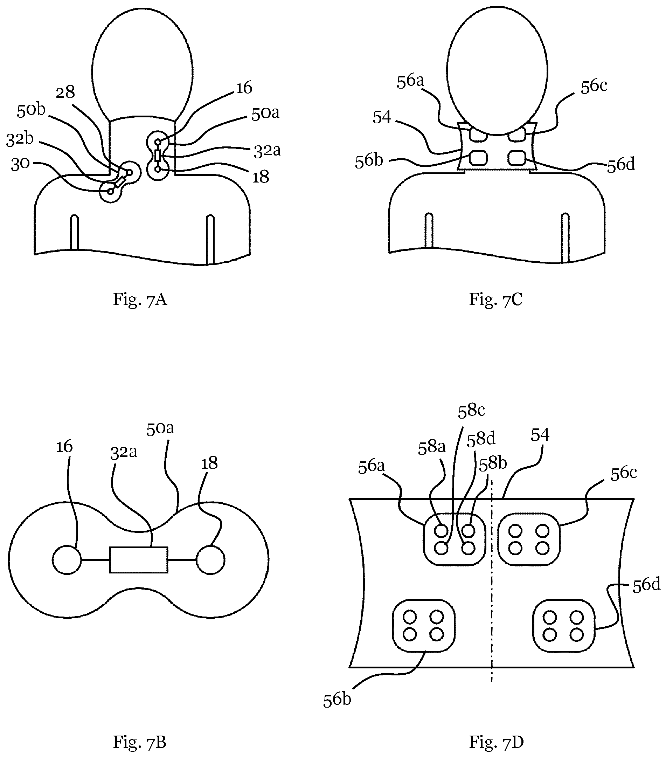

Fig. 7A, 7B illustrates an example of a system 10 for treating cervical dystonia, wherein the system comprises first and second stimulation units 32a, 32b. Each of the first and second stimulation units 32a, 32b is attached to an electrode body 50a, 50b holding first and second electrode contacts 16, 18, 28, 30, of a first and a second electrodes 14, 26, respectively.

The electrode bodies 50a, 50b are configured to adhere to the skin of a patient and may be at least partially flexible such as to follow a surface geometry of the neck of a patient when placed in functional proximity of an agonist muscle 20 or an antagonist muscle 22. The electrode bodies 50a, 50b may hold the first and second electrode contacts 16, 18, 28, 30 at a predetermined spacing, such as to be placed over a target muscle or muscle group 20, 22 and to influence an activity of the target muscle or muscle group 20, 22 by applying pulsed electrical stimulation signals the at least one of the electrode contacts 16, 18, 28, 30.

A specialist may select from a plurality of shapes of individualized electrode bodies 50a, 50b for specific muscles or muscle groups based on the condition and anatomy of the patient, or the electrode body 50a, 50b may be customized for the patient, such as to customize the treatment of a patient using the system 10. For example, the system 10 may comprise a plurality of individualized electrode bodies 50a, 50b with shapes adapted to certain target muscles 20, 22 or patient anatomies, and a subset of the electrode bodies 50a, 50b may be selected by a specialist for a certain form of cervical dystonia and may be employed for the treatment of the patient.

The applied pulsed electrical stimulation signals may be generated in the respective stimulation unit 32a, 32b attached to the electrode body 50a, 50b, and may be transmitted through internal wired connections from the stimulation unit 32a, 32b to the electrode contacts 16, 18, 28, 30 placed in contact with the skin of the patient. As illustrated in Fig. 7B, the stimulation unit 32a may be placed between the first and second electrode contacts 16, 18 on the electrode body 50a to adhere to the skin of the patient with the electrode contacts 16, 18. The stimulation unit 32a, 32b may be attachable to the electrode body 50a, 50b through a connector, such as to enable attachment of the stimulation unit 32a, 32b to a plurality of different individualized electrode bodies 50a, 50b.

Each of the stimulation units 32a, 32b may receive wireless control signals, e.g. from a common control system 24 (not shown), or one of the stimulation units 32a, 32b may assume a main stimulation unit 32a function to communicate with and control operation with the other stimulation unit 32b, e.g. configured as or assuming the function of a secondary stimulation unit 32b-d. In the latter case, the main stimulation unit 32a may communicate with a control system 24 determining the operation parameters of all stimulation units 32a-d.

Fig. 7C, 7D illustrates another example of a system 10 for treating cervical dystonia. The system 10 comprises an electrode support structure 54, depicted as a flexible neck collar, wherein the electrode support structure 54 holds a plurality of electrode pads 56a-d at pre-determined locations of the support structure 54. The support structure 54 may be adapted to the neck of a patient or may be at least partially flexible such as to adjust to the shape of the neck of a patient when worn by the patient. In some embodiments, the support structure 54 may be configured to be mounted to a standard mechanical neck support, e.g. may be formed as an arrangement of padded structures mountable to standard mechanical neck supports.

The geometrical arrangement of the contact pads 56a-d on the support structure 54 may follow a geometry of functional muscle groups 20, 22 of the patient involved in cervical dystonia or may be distributed over the support structure 54 to lie in functional proximity to a plurality of different muscles of the patient involved in cervical dystonia or in a specific cervical dystonia type.

Each electrode pad 56a-d may comprise a plurality of electrode contacts 58a-d for applying electrical stimulation to the skin of the patient wearing the support structure 54 on his neck. For example, the electrode contacts 58a-d of an electrode pad 56a-d may be employed as first and second electrode contacts 16, 18 of a first electrode **14,** or electrode contacts 58a-d of different electrode pads 56a-d may be employed as first and second electrode contacts 16, 18 of a first electrode **14** for applying a pulsed electrical stimulation signal to a target muscle 20, 22 in functional proximity to different electrode pads 56a-d.

The electrode pads 56a-d may feature electric contacts to be connected to a stimulator 12 through wires and/or the stimulator 12 may be configured to selectively apply electrical stimulation signals to a subset of the electrode contacts 58a-d of an electrode pad 56a-d, e.g. by selecting a specific pair of electrode contacts 58a-d of the electrode pad(s) 56a-d and routing electrical stimulation signals to the specific pair of electrode contacts, for selectively increasing or reducing the activity of the agonist muscle 20 or the antagonist muscle 22.

The skilled person will appreciate that the electrode contacts 58a-d are shown with similar size for illustration purposes only and the electrode contacts 58a-d may have different sizes or shapes in embodiments. For example, the support structure 54 may comprise extended contacts for providing a ground/reference potential in a distant location from a target muscle group, such as to enable monopolar operation of the electrode contacts 58a-d.

The stimulation units 32a, 32b or the support structure 54 may be worn by the patient during a training session, such as a training session for a duration of 20 minutes to reduce a deficit of coordination between an agonist muscle 20 and an antagonist muscle 22 through the application of the pulsed electrical stimulation signal. However, if the training proves to be inefficient or the medical condition has already progressed, the electrode(s) 14, 26 may also be implanted in the patient, such as to enable the constant or regular electrical stimulation of the agonist and/or antagonist muscles 20, 22 without visible electrodes **14,** 26 present on the neck of the patient.

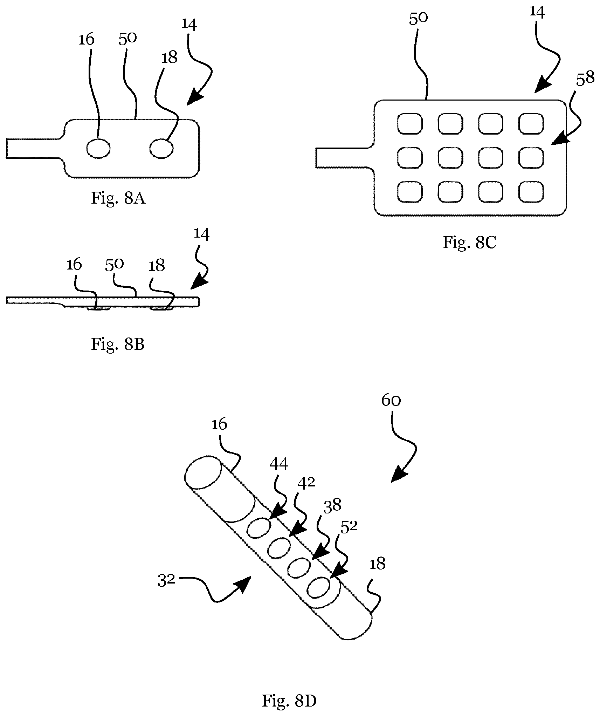

Figs. 8A, 8B illustrate bottom and side views of an implantable first electrode 14 according to an example. The first electrode **14** comprises a first electrode contact 16, and a second electrode contact 18 spaced along an implantable electrode body 50. The implantable electrode body 50 may be flexible to adjust to movements of the electrode body 50 and should be insulating to electrically insulate electrical wires of the first electrode **14** connecting the first electrode contact 16 and the second electrode contact 18 to an implant (not shown).

The first electrode contact 16 and the second electrode contact 18 may be aligned in an in-line geometry and may be accessible and/or protrude from the electrode body 50 on one side of the electrode body 50, while the first electrode contact 16 and the second electrode contact 18 may be spaced along the side of the electrode body 50. The first electrode 14 may be implanted, such that the first electrode contact 16 and the second electrode contact 18 lie in functional proximity of a target muscle (group) 20, 22 (e.g. over or in a target muscle 20, 22), and/or in functional proximity to its innervating nerves, such as to enable (de-)activation of the target muscle(s) 20, 22 with a pulsed stimulation signal applied to the first electrode 14.

The implant may be fixed to the skeleton of the patient, e.g. at the sternum or a collarbone of the patient, and may comprise a stimulator unit 32a-d to generate pulsed electrical stimulation signal(s) for reducing the imbalance in the activity of the muscles 20, 22 of the neck of a patient by one or both of increasing activity of an agonist muscle 20 and reducing activity of an antagonist muscle 22. The implant may be connected to a single electrode 14, or to a plurality of electrodes 14, 26, and may be configured for generating different pulsed electrical stimulation signals to be applied to different electrodes 14, 26.

Although the implantable first electrode 14 is illustrated as a bipolar first electrode 14, different configurations may be used for implantable electrodes 14 in embodiments. For example, the first electrode 14 may comprise a single active contact 16 and may be driven in a monopolar configuration, while a ground/reference potential may be applied at a larger contact surface, e.g. a ground contact of the implant. In addition, the first electrode 14 may comprise a tripolar or multipolar configuration, e.g. may be configured as a cuff electrode to at least partially wrap around target tissue, e.g. a target muscle 20, 22 or an innervating nerve.

Figure 8C illustrates another example of an implantable first electrode 14, wherein the first electrode 14 comprises an array of electrode contacts 58. In the illustrated example, the electrode contacts of the array of electrode contacts 58 are distributed in a regular matrix to be connected to an implant (not shown). The illustrated array of electrode contacts is arranged in a square matrix to enable electrical stimulation of the target tissue at different locations in functional proximity of the electrode body 50.

The implant may be configured to select a subset of electrode contacts among the plurality of electrode contacts of the array of electrode contacts 58 to apply a pulsed electrical stimulation signal to a target tissue portion of the electrode array. For example, the first electrode 14 with the array of electrode contacts 58 may be implanted in a patient and a subset of electrode contacts may be selected based on a muscle response of the patient such as to determine optimal contacts for stimulation of the target muscle 20, 22.

In some embodiments, the implant may be configured to alternatingly apply the pulsed electrical stimulation signal to different subset of electrode contacts, such as to reduce muscle fatigue or compensate movement of the patient.

Fig. 8D illustrates an example of an implant 60, wherein the stimulator unit 32a is arranged between first and second contacts 16, 18 of the implant 60. The implant 60 comprises a power source 42, a communication interface 38, a control unit 44 and a stimulation control unit 52. The implant 60 may constitute a (self-sufficient) treatment system 10 or may be driven as a secondary stimulation unit 32b-d by a main stimulation unit 32a in another implant (not shown) of a system 10 with multiple stimulation units 32a-d.

The power source 42 may comprise a battery and power management circuit to distribute power between the components 38, 44, 52 of the implant 60 and to receive power, e.g. inductively from a non-implantable component of the implant 60, or through wired connections to a main stimulation unit 32a in another implant.

The communication interface 38 may be configured to receive control instructions from a main stimulation unit 32a or a control system 24 through a wireless communication pathway. The control device 44 may process control instructions from the communication interface 38 and may control the stimulation control unit 52 based on the control instructions.

The stimulation control unit 52 may generate pulsed electrical stimulation signals and may be connected to the electrode contacts 16, 18 of the implant 60 to apply pulsed electrical stimulation signals to an agonist or antagonist muscle 20, 22 in functional proximity to the implant 60.

As illustrated in Fig. 8C, the electrode contacts 16, 18 may be provided on opposite sides of the implant 60, such that the implant 60 may be implanted in or over a target muscle 20, 22 to provide an integrated electrode and stimulation system 10 in a compact implantable package. However, the skilled person will appreciate that the electrode contacts 16, 18 may equally be spaced from the implant 60, e.g. as an implantable first electrode 14 in an electrode body 50 as illustrated in Figs. 8A-8C, in embodiments.

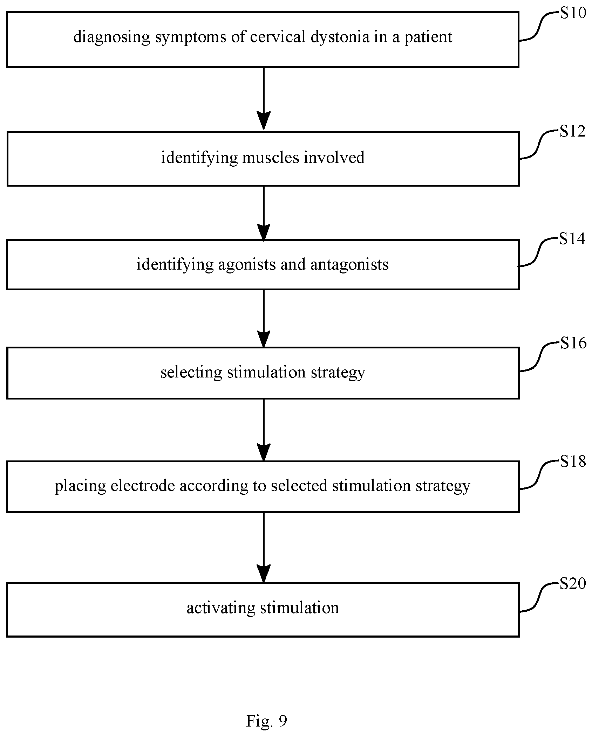

Fig. 9 shows a schematic flowchart of a treatment method for treating cervical dystonia according to an example. The method comprises diagnosing symptoms of cervical dystonia in a patient (S10), and identifying the muscles involved (S12) in the condition. The method further comprises agonist 20 and antagonist muscles 22 (S14) and selecting a stimulation strategy (S16). The method then comprises placing at least one electrode 14, 26 according to the selected stimulation strategy (S18) and activating the stimulation through the at least one electrode 14, 26 (S20).

Diagnosing symptoms (S10) may include, but is not limited to determining whether the chin is twisted towards a shoulder so that the head rotates sideways; or whether the head tips forward; or whether the head is tilted backward; or whether the head tilts toward one side; or whether the head is shifted on the shoulders in a forward (anterior sagittal shift) or backward (posterior sagittal shift) direction.

In a next step a specialist may identify at least some of the muscles involved (S12) causing these symptoms. In general, any of the muscles: musculus sternocleidomastoideus; musculus trapezius; musculus scaleni medius et posterior; musculus splenius capitis; musculus levator scapulae; musculus semispinalis capitis et cervicis; musculus splenii capitis et cervicis; musculus digastricus; musculus omohyoideus; platysma; musculus longus colli et capitis; musculus rhomboidei minor et major; musculus longisimus capitis et cervicis; musculus rectus lateralis; musculus rectus capitis posterior minor et major; musculus obliquus capitis superior et inferior; and musculus scalene complex may be involved. The skilled person will appreciate that all these muscles are present twice, i.e. on each side (left and right) of the sagittal plane. As an example, the M. sternocleidomastoideus comprises a left and a right M. sternocleidomastoideus, or, differently stated, an ipsilateral and a contralateral M. sternocleidomastoideus. Determining muscles involved may include but is not limited to visual, EMG, electroneuronography (ENog) examinations.

Based on the preceding two steps (S10, S12) the specialist may identify which muscles show hypo-functional activity and therefore act as agonists 20 and which show hyper-functional activity and therefore act as antagonists 22 in this patient (S14).

Based on these diagnosis steps the specialist may determine the stimulation strategy (S16). The stimulation strategy may determine the muscles and/or innervating nerves to be stimulated, whether both agonist(s) and antagonist(s) will be stimulated and whether they are stimulated simultaneously or sequentially.

For example, the stimulation strategy may comprise the simultaneous application of electrostimulation to activate/increase the hypo-functional agonistic activity and to reduce hyper-functional antagonistic activity. As another example, the stimulation strategy may comprise the sequential application of electrostimulation to activate/increase the hypo-functional agonistic activity and to reduce hyper-functional antagonistic activity. Further, the stimulation strategy may comprise the application of electrostimulation to activate/increase the hypo-functional agonistic activity without stimulation to reduce the hyper-functional antagonistic activity, or the application of electrostimulation to reduce the hyper-functional antagonistic activity without stimulation to activate/increase hypo-functional agonistic activity.

The method may then comprise the determination of the placement of the stimulation electrodes 14, 26 (S18). In general stimulation electrodes are placed in functional proximity to the muscles 20, 22and/or their innervating nerves intended to be stimulated.

For example, the placement may comprise electrode arrangement(s), such as, without limitation, pairs of electrode contacts 16, 18 if the stimulation is provided in a bipolar mode, triples of electrode contacts 16, 18 if the stimulation is provided in tripolar mode (this arrangement especially in connection with cuff electrodes, see below), or single electrode contacts 16 if the stimulation is provided in mono-polar mode. In the latter case a reference electrode 18 may be placed elsewhere.

In the case of application of electrostimulation to reduce the hyper-functional antagonistic activity without stimulating hypo-functional agonistic activity, electrodes 14, 26 may be placed in functional proximity to one or more muscles showing hyper-functional antagonistic activity.

In the case of application of electrostimulation to activate/increase the hypo-functional agonistic activity without stimulating hyper-functional antagonistic activity, stimulation electrodes 14, 26 may be placed in functional proximity to at least one, to some or to all of the agonistic muscles.

In the case of application of electrostimulation to activate/increase the hypo-functional agonistic activity and to reduce hyper-functional antagonistic activity, stimulation electrodes 14, 26 may be placed in functional proximity to at least one, to some or to all of the relevant agonist muscles 20 and antagonist muscles 22, respectively, but at least to one muscles 20 showing hypo-functional agonistic activity and to one muscles 22 showing hyper-functional antagonistic activity.

The electrical pulses may be provided simultaneously or sequentially via the electrodes 14, 26 selected for and used in a specific patient. E.g., the electrical pulses may be delivered to all the agonist muscles 20 and to all these antagonist muscles 22 simultaneously, i.e. all the pulses are provided at the same time. If pulse durations are different, then the pulses may have a common timely overlap.

In another embodiment the electrical pulses may be delivered to all these agonist muscles 20 and to all these antagonist muscles 22 sequentially, e.g. without an overlap in time between pulses delivered to the agonist muscles 20 and the antagonist muscles 22 of relevance.

In preferred embodiments, electrical pulses are provided to all these agonist muscles 20 simultaneously and to all these antagonist muscles 22 simultaneously, but sequentially between all the involved agonist muscles 20 and all these antagonist muscles 22.

However, in principle any combination of sequential and simultaneous stimulation among all muscles 20, 22 may be applied.

Irrespective of the selected combination of sequential and simultaneous stimulation, agonist muscles 20 (addressing hypo-active muscles) are preferably stimulated using biphasic pulses and antagonist muscles 22 (addressing hyper-active muscles) are preferably stimulated using triphasic pulses.

The stimulation may then be activated (S20), e.g. by actuating an activating switch, and the target tissue may be stimulated according to the selected stimulation strategy and via the electrodes 14, 26 placed according to the selected stimulation strategy.

In the following some specific examples are provided for torticollis, laterocollis, anterocollis, and retrocollis.

Torticollis is the most common abnormal posture associated with cervical dystonia. Patients suffering from torticollis have their chin twisted toward a shoulder so that the head rotates sideways.

In most of these cases the musculus sternocleidomastoideus at the ipsilateral side (i.e. the side to which the chin is twisted) is the agonist muscle 20 and shows hypo-functional activity. Accordingly, an electrode arrangement 14 is placed in functional proximity to this muscle 20 and biphasic stimulation pulses are applied to this muscle 20 and/or innervating nerves via this electrode arrangement 14.

In addition, the specialist may also identify at least one muscle 22, that shows hyper-active function. This at least one muscle 22 may be e.g. the contralateral sternocleidomastoideus. Accordingly, the specialist may place an electrode arrangement 26 in functional proximity to this contralateral muscle 22 and triphasic stimulation pulses may be applied to this muscle 22 and/or innervating nerves via this electrode arrangement 26.

Alternatively or in addition to the above, the specialist may also identify at least one of the muscles 22 splenius capitis and semispinalis capitis, both at the ipsilateral side, showing antagonistic functionality. Accordingly, the specialist may place electrode arrangements 26 in functional proximity to the ipsilateral antagonist muscles 22 and/or their nerves and triphasic stimulation pulses may be applied to the muscles 22 and/or their innervating nerves via the electrode arrangements 26.

**In** laterocollis, the head tilts toward one side.

**In** most of these cases the musculus sternocleidomastoideus at the ipsilateral side (i.e. the side to which the head is tilted) is the agonist muscle 20 and shows hypo-functional activity. Accordingly, an electrode arrangement **14** is placed in functional proximity to this muscle 20 and biphasic stimulation pulses may be applied to this muscle 20 and/or innervating nerves via this electrode arrangement **14.**

**In** addition, the specialist may also identify at least one muscle 22 that shows hyper-active function. This at least one muscle 22 may be e.g. the contralateral sternocleidomastoideus. Accordingly, the specialist may place an electrode arrangement 26 in functional proximity to this contralateral muscle 26 and triphasic stimulation pulses may be applied to this muscle 22 and/or its innervating nerves via the electrode arrangement 26.

Alternatively or in addition to the above, the specialist may also identify at least one of the muscles 22 splenius capitis, semispinalis capitis and levator scapulae, all three at the ipsilateral side, showing antagonistic functionality. Accordingly, the specialist may place electrode arrangements 26 in functional proximity to these ipsilateral muscles 22 and triphasic stimulation pulses may be applied to this muscle 22 and/or its innervating nerves via the electrode arrangements 26.

**In** anterocollis, the head tips forward.

**In** most of these cases the musculus sternocleidomastoideus (both ipsi- and contralateral) is the agonist 20 and shows hypo-functional activity. Accordingly, electrode arrangements **14** may be placed in functional proximity to these muscles 20 and biphasic stimulation pulses may be applied to these muscles 20 and/or innervating nerves via the electrode arrangement **14.**

Alternatively or in addition to the above, the specialist may also identify the musculus scalene complex (both ipsi- and contralateral) showing agonistic functionality. Accordingly, the specialist may place electrode arrangements 26 in functional proximity to these muscles 20 and biphasic stimulation pulses may be applied to these muscles 20 and/or innervating nerves via the electrode arrangements 26.

**In** addition, the specialist may also identify at least one muscle 22, that shows hyper-active function. This at least one muscle 22 may be e.g. the trapezius pars superior (both ipsi- and contralateral). Accordingly, the specialist may place electrode arrangements 26 in functional proximity to this contralateral muscle 22 and triphasic stimulation pulses may be applied to this muscle 22 and/or innervating nerves via the electrode arrangement 26.

Alternatively or in addition to the above, the specialist may also identify at least one of the muscles splenius capitis and semispinalis capitis showing antagonistic functionality (both ipsi- and contralateral). Accordingly, the specialist may place electrode arrangements 26 in functional proximity to these ipsilateral muscles 22 and triphasic stimulation pulses may be applied to these muscles 22 and/or innervating nerves via the electrode arrangements 26.

**In** retrocollis, the head tilts backward.

**In** most of these cases the trapezius pars superior (both ipsi- and contralateral) is the agonist muscle 20 and shows hypo-functional activity. Accordingly, an electrode arrangement **14** may be placed in functional proximity to this muscle 20 and biphasic stimulation pulses may be applied to this muscle 20 and/or its innervating nerves via this electrode arrangement **14.**

Alternatively or in addition to the above, the specialist may also identify at least one of the muscles splenius capitis and semispinalis capitis (both ipsi- and contralateral) showing agonistic functionality. Accordingly, the specialist may place electrode arrangements **14 in** functional proximity to these muscles 20 and biphasic stimulation pulses may be applied to the muscles 20 and/or innervating nerves via these electrode arrangements **14.**

**In** addition, the specialist may also identify at least one muscle 22, that shows hyper-active function. This at least one muscle may be e.g. sternocleidomastoideus (both ipsi- and contralateral). Accordingly, the specialist may place electrode arrangements 26 in functional proximity to these muscles 22 and triphasic stimulation pulses may be applied to these muscles 22 and/or innervating nerves via the electrode arrangements 26.

Alternatively or in addition to the above, the specialist may also identify the musculus scalene complex showing antagonistic functionality (both ipsi- and contralateral). Accordingly, the specialist may place electrode arrangements 26 in functional proximity to these muscles 22 and triphasic stimulation pulses may be applied to the muscles 22 and/or their innervating nerves via the electrode arrangements 26.

A stimulation system 10 may either be non-implantable or implantable.

For example, the specialist may instruct the patient to place the electrodes 14, 26 superficially on the skin of the neck such that they are overlying target tissue. The electrodes 14, 26 may be glued to the skin, e.g. via an adhesive coating on an electrode body 50. In addition or alternatively, the electrodes 14, 26 may be physically connected to a support structure 54 like a neck brace. For example, a suitable electrode arrangement for a support structure 54 may be selected based on the stimulation strategy and may be worn by the patient for therapeutic purposes.

Patients may use the stimulation system 10 for therapeutic purposes twice to three times per day for about 30 min per session. After some therapeutic sessions, in many cases patients will have relief from their symptoms. In this case a non-implantable stimulation system 10 may be sufficient.

If the stimulation, however, is needed all or most of the time during the day, then an implantable stimulation system 10 may be preferred. The electrodes 14, 26 can then be invisible and thus it may be easier for the patients to wear them during social events.

At least one stimulation unit 32, 32a-d may be implanted into the body as an implant 60 and may be fixed e.g. at or near the sternum or at one of the collarbones by a specialist.

The electrode arrangements 14, 26, 58 may be implantable electrodes 14, 26 and may brought into direct contact with target tissue e.g. by fixating them in, around or just attached to target tissue e.g. as rod electrodes, cuff electrodes, etc. Single electrode contacts 16 may be used in mono-polar stimulation mode and a reference electrode 18 may be provided separately somewhere in the neck area or on the outside of the stimulation unit 32, 32a-d, e.g. on a body of the implant 60.

Both in implantable and non-implantable stimulation systems electrical pulses may be generated in the stimulation unit by one or more pulse generators and transmitted via one or more electrically conductive wires to the one or more electrodes which in turn forward the electrical pulses to target tissue.

The operation parameters for the pulse generators may be selected by the specialist and/or may be reconfigurable through a wired/wireless interface to the stimulation unit 32, 32a-d to provide a versatile stimulation system 10.

The description of the preferred embodiments and the figures merely serve to illustrate the invention and the beneficial effects associated therewith, but should not be understood to imply any limitation. The scope of the invention is to be determined solely by the appended claims.

### LIST OF REFERENCE SIGNS

- 10: system
- 12: stimulator
- **14**: first electrode
- 16: first electrode contact of the first electrode
- 18: second electrode contact of the first electrode
- 20: agonist muscle
- 22: antagonist muscle
- 24: control system
- 26: second electrode
- 28: first electrode contact of the second electrode
- 30: second electrode contact of the second electrode
- 32: stimulation unit
- 32a: main stimulation unit
- 32b-d: secondary stimulation units
- 34: user interface
- 36: communication interface of the control system
- 38, 38a,b: communication interface of the stimulation unit
- 40: power interface
- 42, 42a, b: power source
- **44**: control device
- 46: connection interface
- 48a, b: internal/external sensor
- 50: electrode body
- 52, 52a, b: stimulation control unit
- 54: support structure
- 56a-d: electrode pads
- 58: electrode array
- 58a-d: electrode contacts
- 60: implant

## Claims

1. A system (10) for treating cervical dystonia, wherein the system (10) comprises
a first electrode (14) adapted to be placed in functional proximity to at least one of an agonist muscle (20) and an antagonist muscle (20, 22) and/or their innervating nerves,
a stimulator coupled to the first electrode (14) and configured to apply a pulsed electrical stimulation signal to the first electrode (14), wherein the pulsed electrical stimulation signal is configured for selectively increasing or reducing the activity of the at least one of the agonist muscle (20) and the antagonist muscle (20, 22),
wherein the pulsed electrical stimulation signal is adapted to reduce an imbalance in the activity of the agonist muscle (20) and the antagonist muscle (22), wherein the pulsed electrical stimulation signal is configured for reducing the imbalance in the activity of the agonist muscle (20) and the antagonist muscle (20, 22) by one or both of increasing activity of the agonist muscle (20) using a biphasic electrical stimulation pulse and reducing activity of the antagonist muscle (20, 22) using a triphasic electrical stimulation pulse.

2. The system (10) of claim 1, wherein the electrical stimulation pulse is adapted to stimulate super-threshold.

3. The system (10) of claim 1 or 2, wherein the electrical stimulation pulse is charge-balanced.

4. The system (10) of claim 1 or 2, wherein the agonist muscle (20) and/or the antagonist muscle (22) are selected from the group of: musculus sternocleidomastoideus; musculus trapezius; musculus scaleni medius et posterior; musculus splenius capitis; musculus levator scapulae; musculus semispinalis capitis et cervicis; musculus splenii capitis et cervicis; musculus digastricus; musculus omohyoideus; platysma; musculus longus colli et capitis; musculus rhomboidei minor et major; musculus longisimus capitis et cervicis; musculus rectus lateralis; musculus rectus capitis posterior minor et major; musculus obliquus capitis superior et inferior; and musculus scalene complex.

5. The system (10) of claim 1, wherein the pulsed electrical stimulation signal has a frequency or amplitude modulated signal envelope and the amplitude modulated signal envelope has a rectangular or sine signal envelope.

6. The system (10) of any one of claims 1 to 5,
wherein the first electrode (14) is placed or adapted to be placed on the agonist muscle (20) and/or its innervating nerve, and
wherein the system (10) comprises a second electrode (26),
wherein the second electrode (26) is placed or adapted to be placed on the antagonist muscle (20, 22) and/or its innervating nerve,
wherein the pulsed electrical stimulation signal comprises a first pulsed electrical stimulation signal and a second pulsed electrical stimulation signal, and
wherein the stimulator is configured to apply the first pulsed electrical stimulation signal to the first electrode (14) and to apply the second pulsed electrical stimulation signal to the second electrode (26) to reduce the imbalance in the activity of the agonist muscle (20) and the antagonist muscle (22).

7. The system (10) of claim 6, wherein at least one of the first and second electrodes (14, 26) comprises a first electrode contact (16, 28) and a second electrode contact (18, 30), wherein the first electrode contact (16, 28) and the second electrode contact (18, 30) are connected to a respective first wire and a second wire to stimulator (12), and wherein the stimulator (12) is configured to apply a pulsed electrical stimulation signal of opposite polarity to the first electrode contact (16, 28) and the second electrode (18, 30) contact through the first wire and the second wire, respectively, and/or wherein the stimulator (12) is configured to apply a pulsed electrical stimulation signal to the first electrode contact (16, 28), while the second electrode contact (18, 30) is grounded.

8. The system (10) of any one of the preceding claims, wherein at least one of the first electrode (14) and the second electrode (26) comprises a plurality of electrode contacts (16, 18, 28, 30), wherein the stimulator is configured to select a subset of at least two electrode contacts (16, 18, 28, 30) among the plurality of electrode contacts (16, 18, 28, 30) for applying the pulsed electrical stimulation signal.

9. The system (10) of any one of the preceding claims, wherein the stimulator (12) is configured to apply the pulsed electrical stimulation signal with a duty cycle comprising a high intensity cycle and a low intensity cycle, wherein the stimulator (12) switches between the high intensity cycle and the low intensity cycle in regular time intervals, wherein a duration of the high intensity cycle and the low intensity cycle is in particular smaller than 10 ms, and wherein a duration of the high intensity cycle is in particular larger than a duration of the low intensity cycle.

10. The system (10) of any one of the preceding claims, wherein the system (10) further comprises a sensor (48a, 48b) to detect an activity of the agonist muscle (20) and/or the antagonist muscle (22).

11. The system (10) of any one of the preceding claims, wherein the first electrode (14) is an implantable electrode, and wherein the system (10) comprises an implant (60) which includes the stimulator.

## Patentansprüche

1. System (10) zur Behandlung von Zervixdystonie, wobei das System (10) Folgendes umfasst:
eine erste Elektrode (14), die dafür ausgelegt ist, in funktioneller Nähe zu einem Agonistenmuskel (20) und/oder einem Antagonistenmuskel (20, 22) und/oder deren innervierenden Nerven platziert zu werden,
einen Stimulator, der mit der ersten Elektrode (14) gekoppelt ist und dafür ausgelegt ist, ein gepulstes elektrisches Stimulationssignal an die erste Elektrode (14) anzulegen, wobei das gepulste elektrische Stimulationssignal dafür ausgelegt ist, die Aktivität des Agonistenmuskels (20) und/oder des Antagonistenmuskels (20, 22) selektiv zu erhöhen oder zu verringern,
wobei das gepulste elektrische Stimulationssignal dafür ausgelegt ist, ein Ungleichgewicht in der Aktivität des Agonistenmuskels (20) und des Antagonistenmuskels (22) zu verringern, wobei das gepulste elektrische Stimulationssignal dafür ausgelegt ist, das Ungleichgewicht in der Aktivität des Agonistenmuskels (20) und des Antagonistenmuskels (20, 22) durch Erhöhen der Aktivität des Agonistenmuskels (20) unter Verwendung eines zweiphasigen elektrischen Stimulationsimpulses und/oder Verringern der Aktivität des Antagonistenmuskels (20, 22) unter Verwendung eines dreiphasigen elektrischen Stimulationsimpulses zu verringern.

2. System (10) nach Anspruch 1, wobei der elektrische Stimulationsimpuls dafür ausgelegt ist, einen Superschwellenwert zu stimulieren.

3. System (10) nach Anspruch 1 oder 2, wobei der elektrische Stimulationsimpuls ladungsausgeglichen ist.

4. System (10) nach Anspruch 1 oder 2, wobei der Agonistenmuskel (20) und/oder der Antagonistenmuskel (22) aus der folgenden Gruppe ausgewählt sind: Musculus sternocleidomastoideus; Musculus trapezius; Musculus scaleni medius et posterior; Musculus splenius capitis; Musculus levator scapulae; Musculus semispinalis capitis et cervicis; Musculus splenii capitis et cervicis; Musculus digastricus; Musculus omohyoideus; Platysma; Musculus longus colli et capitis; Musculus rhomboidei minor et major; Musculus longisimus capitis et cervicis; Musculus rectus lateralis; Musculus rectus capitis posterior minor et major; Musculus obliquus capitis superior et inferior; und Musculus scalens complex.

5. System (10) nach Anspruch 1, wobei das gepulste elektrische Stimulationssignal eine frequenz- oder amplitudenmodulierte Signalhüllkurve aufweist und die amplitudenmodulierte Signalhüllkurve eine Rechteck- oder Sinussignalhüllkurve aufweist.

6. System (10) nach einem der Ansprüche 1 bis 5,
wobei die erste Elektrode (14) auf dem Agonistenmuskel (20) und/oder seinem innervierenden Nerv platziert ist oder dafür ausgelegt ist, darauf platziert zu werden, und
wobei das System (10) eine zweite Elektrode (26) umfasst,
wobei die zweite Elektrode (26) auf dem Antagonistenmuskel (20, 22) und/oder seinem innervierenden Nerv platziert ist oder dafür ausgelegt ist, darauf platziert zu werden,
wobei das gepulste elektrische Stimulationssignal ein erstes gepulstes elektrisches Stimulationssignal und ein zweites gepulstes elektrisches Stimulationssignal umfasst, und
wobei der Stimulator dafür ausgelegt ist, das erste gepulste elektrische Stimulationssignal an die erste Elektrode (14) anzulegen und das zweite gepulste elektrische Stimulationssignal an die zweite Elektrode (26) anzulegen, um das Ungleichgewicht in der Aktivität des Agonistenmuskels (20) und des Antagonistenmuskels (22) zu verringern.

7. System (10) nach Anspruch 6, wobei die erste und/oder die zweite Elektrode (14, 26) einen ersten Elektrodenkontakt (16, 28) und einen zweiten Elektrodenkontakt (18, 30) umfasst, wobei der erste Elektrodenkontakt (16, 28) und der zweite Elektrodenkontakt (18, 30) mit einem jeweiligen ersten Draht und einem zweiten Draht zu dem Stimulator (12) verbunden sind und wobei der Stimulator (12) dafür ausgelegt ist, ein gepulstes elektrisches Stimulationssignal mit entgegengesetzter Polarität an den ersten Elektrodenkontakt (16, 28) und den zweiten Elektrodenkontakt (18, 30) durch den ersten Draht bzw. den zweiten Draht anzulegen und/oder wobei der Stimulator (12) dafür ausgelegt ist, ein gepulstes elektrisches Stimulationssignal an den ersten Elektrodenkontakt (16, 28) anzulegen, während der zweite Elektrodenkontakt (18, 30) geerdet ist.

8. System (10) nach einem der vorhergehenden Ansprüche, wobei die erste Elektrode (14) und/oder die zweite Elektrode (26) mehrere Elektrodenkontakte (16, 18, 28, 30) umfasst, wobei der Stimulator dafür ausgelegt ist, eine Teilmenge von mindestens zwei Elektrodenkontakten (16, 18, 28, 30) aus den mehreren Elektrodenkontakten (16, 18, 28, 30) zum Anlegen des gepulsten elektrischen Stimulationssignals auszuwählen.

9. System (10) nach einem der vorhergehenden Ansprüche, wobei der Stimulator (12) dafür ausgelegt ist, das gepulste elektrische Stimulationssignal mit einem Arbeitszyklus anzulegen, der einen Hochintensitätszyklus und einen Niedrigintensitätszyklus umfasst, wobei der Stimulator (12) in regelmäßigen Zeitintervallen zwischen dem Hochintensitätszyklus und dem Niedrigintensitätszyklus umschaltet, wobei eine Dauer des Hochintensitätszyklus und des Niedrigintensitätszyklus insbesondere kleiner als 10 ms ist und wobei eine Dauer des Hochintensitätszyklus insbesondere größer als eine Dauer des Niedrigintensitätszyklus ist.

10. System (10) nach einem der vorhergehenden Ansprüche, wobei das System (10) ferner einen Sensor (48a, 48b) zum Erfassen einer Aktivität des Agonistenmuskels (20) und/oder des Antagonistenmuskels (22) umfasst.

11. System (10) nach einem der vorhergehenden Ansprüche, wobei die erste Elektrode (14) eine implantierbare Elektrode ist und wobei das System (10) ein Implantat (60) umfasst, das den Stimulator enthält.

## Revendications

1. Système (10) pour le traitement de la dystonie cervicale, le système (10) comprenant une première électrode (14) placée ou apte à être placée à proximité fonctionnelle d'au moins un muscle agoniste (20) et/ou d'un muscle antagoniste (20, 22) et/ou de leurs nerfs innervants, un stimulateur couplé à la première électrode (14) et configuré pour appliquer un signal de stimulation électrique pulsée à la première électrode (14), ledit signal étant configuré pour augmenter ou réduire sélectivement l'activité du muscle agoniste (20) et/ou du muscle antagoniste (20, 22), pour réduire un déséquilibre d'activité entre ces muscles, et pour réduire ce déséquilibre en augmentant l'activité du muscle agoniste (20) au moyen d'une impulsion biphasique et/ou en réduisant l'activité du muscle antagoniste (20, 22) au moyen d'une impulsion triphasique.

2. Système (10) selon la revendication 1, dans lequel l'impulsion de stimulation électrique est supraliminaire.

3. Système (10) selon la revendication 1 ou 2, dans lequel l'impulsion est équilibrée en charge.

4. Système (10) selon la revendication 1 ou 2, dans lequel le muscle agoniste (20) et/ou le muscle antagoniste (22) sont sélectionnés dans le groupe comprenant : musculus sternocleidomastoideus ; musculus trapezius ; musculus scaleni medius et posterior ; musculus splenius capitis ; musculus levator scapulae ; musculus semispinalis capitis et cervicis ; musculus splenii capitis et cervicis ; musculus digastricus ; musculus omohyoideus ; platysma ; musculus longus colli et capitis ; musculus rhomboidei minor et major ; musculus longisimus capitis et cervicis ; musculus rectus lateralis ; musculus rectus capitis posterior minor et major ; musculus obliquus capitis superior et inferior ; et musculus scalene complex.

5. Système (10) selon la revendication 1, dans lequel le signal de stimulation électrique pulsée présente une enveloppe modulée en fréquence ou en amplitude, et dans lequel l'enveloppe de signal modulée en amplitude présente une enveloppe rectangulaire ou sinusoïdale.

6. Système (10) selon l'une quelconque des revendications 1 à 5,
dans lequel la première électrode (14) est placée ou apte à être placée sur le muscle agoniste (20) et/ou sur son nerf innervant,
et dans lequel le système (10) comprend une seconde électrode (26),
dans lequel la seconde électrode (26) est placée ou apte à être placée sur le muscle antagoniste (20, 22) et/ou sur son nerf innervant,
dans lequel le signal de stimulation électrique pulsée comprend un premier signal de stimulation électrique pulsée et un second signal de stimulation électrique pulsée,
et dans lequel le stimulateur est configuré pour appliquer le premier signal de stimulation électrique pulsée à la première électrode (14) et pour appliquer le second signal de stimulation électrique pulsée à la seconde électrode (26) afin de réduire le déséquilibre dans l'activité du muscle agoniste (20) et du muscle antagoniste (22).

7. Système (10) selon la revendication 6,
dans lequel au moins l'une de la première électrode et de la seconde électrode (14, 26) comprend un premier contact d'électrode (16, 28) et un second contact d'électrode (18, 30),
dans lequel le premier contact d'électrode (16, 28) et le second contact d'électrode (18, 30) sont reliés respectivement à un premier fil et à un second fil au stimulateur (12),
et dans lequel le stimulateur (12) est configuré pour appliquer un signal de stimulation électrique pulsée de polarité opposée au premier contact d'électrode (16, 28) et au second contact d'électrode (18, 30) via le premier fil et le second fil, respectivement,
et/ou dans lequel le stimulateur (12) est configuré pour appliquer un signal de stimulation électrique pulsée au premier contact d'électrode (16, 28), tandis que le second contact d'électrode (18, 30) est mis à la masse.

8. Système (10) selon l'une quelconque des revendications précédentes,
dans lequel au moins l'une de la première électrode (14) et de la seconde électrode (26) comprend une pluralité de contacts d'électrode (16, 18, 28, 30),
et dans lequel le stimulateur est configuré pour sélectionner un sous-ensemble d'au moins deux contacts d'électrode (16, 18, 28, 30) parmi ladite pluralité de contacts d'électrode (16, 18, 28, 30) pour l'application du signal de stimulation électrique pulsée.

9. Système (10) selon l'une quelconque des revendications précédentes,
dans lequel le stimulateur (12) est configuré pour appliquer le signal de stimulation électrique pulsée avec un cycle de service comprenant un cycle de haute intensité et un cycle de basse intensité,
dans lequel le stimulateur (12) commute entre le cycle de haute intensité et le cycle de basse intensité à des intervalles de temps réguliers,
dans lequel une durée du cycle de haute intensité et du cycle de basse intensité est en particulier inférieure à 10 ms,
et dans lequel une durée du cycle de haute intensité est en particulier supérieure à une durée du cycle de basse intensité.

10. Système (10) selon l'une quelconque des revendications précédentes, dans lequel le système (10) comprend en outre un capteur (48a, 48b) configuré pour détecter une activité du muscle agoniste (20) et/ou du muscle antagoniste (22).

11. Système (10) selon l'une quelconque des revendications précédentes, dans lequel la première électrode (14) est une électrode implantable, et dans lequel le système (10) comprend un implant (60) qui inclut le stimulateur.
